# EUROPEAN PATENT APPLICATION

(11) **EP 4 527 933 A1**
(43) Date of publication of application: **26.03.2025**
(21) Application number: 22942549.1
(22) Date of filing: 30.12.2022
(51) Int. Cl.: C12N 15/864, C12N 15/67, C12N 15/12, A61K 48/00, A61K 38/17, A61P 27/16

(54) **DUAL-CARRIER SYSTEM FOR TREATING HEARING DAMAGE, AND USE THEREOF**

(30) Priority: 17.05.2022 CN 202210536327
(71) Applicant: Eye & Ent Hospital Of Fudan University, Shanghai 200031 (CN); SHANGHAI REFRESHGENE THERAPEUTICS CO., LTD., Shanghai 201131 (CN)
(72) Inventor: SHU, Yilai, Shanghai 200031 (CN); LI, Huawei, Shanghai 200031 (CN); ZHOU, Rui, Shanghai 201131 (CN); TAN, Qingqiao, Shanghai 201131 (CN); TANG, Honghai, Shanghai 200031 (CN); WANG, Hui, Shanghai 200031 (CN); GAO, Kaiyu, Shanghai 201131 (CN)
(74) Representative: Bayramoglu et al.
(86) International application number: PCT/CN2022/144017
(87) International publication number: WO 2023/221530

(57) **Abstract**

The present invention belongs to the field of gene therapy in the medical category, and particularly relates to use in restoring, by using overexpression of a normal gene, hearing of a patient with hereditary hearing loss caused by gene mutation or deficiency. The present invention relates to a dual-carrier system for expressing an OTOF protein. The dual-carrier system comprises two nucleotide sequences: a first nucleotide sequence comprising two ITR sequences and a gene expression cassette inserted between the ITR sequences; and a second nucleotide sequence comprising two ITR sequences and a gene expression cassette inserted between the ITR sequences. Meanwhile, provided is an adeno-associated virus packaged in the above carrier. The above carrier and virus can restore binaural hearing by means of uniaural administration in the field of large-gene dual-carrier delivery in deafness gene therapy.

## Description

### TECHNICAL FIELD

The present disclosure belongs to the field of gene therapy in the medical field and particularly relates to use of an overexpression of a normal gene in restoring hereditary hearing loss caused by gene mutation or deletion.

### BACKGROUND

The ear is an important organ of human body and consists of the external, middle and inner ear. Its main function is to sense sound and maintain physical balance. When the ear functions abnormally, a series of pathological changes of body functions can be caused, including deafness, tinnitus, dizziness and the like.

Deafness is a common disorder with abnormal auditory functions and can be classified into congenital deafness and acquired deafness. The deafness is mostly related to genetic and environmental factors. There are 2 congenital deafness in 1,000 newborn babies, of which the deafness of 50-60% of patients is caused by gene mutation. The deafness caused by the gene mutation can be classified into dominant gene mutation and recessive gene mutation. Dominant deafness genes comprise *ACTG1, CCDC50, CD164, CEACAM16, DIAPH1* and the like. Recessive deafness genes comprise *CLDN14, PJVK, GRXCR1, MYO7A, MYO6, MYO3A, MYO15A, OTOF, OTOG, OTOA, STRC, TMC1, SLC22A4, SLC26A4, SLC26A5, TECTA, GJB2, GJB6* and the like. The discovery of these deafness genes provides potential targets for accurate treatment of hereditary deafness. Therefore, when the above genes trigger deafness, gene therapy can be used as a first-choice strategy for radically treating the deafness.

The gene therapy refers to a method for achieving a therapeutic object by correcting, compensating or inhibiting at a DNA or RNA level, thereby restoring a disease caused by abnormal nucleic acid sequence or expression *in vivo.* Currently, most gene therapy requires vector delivery. Adeno-associated viruses (AAVs) are safe and efficient delivery vectors with packaging capacity of about 4.7 Kb. However, in the field of deafness, coding regions of many genes are not suitable for packaging adeno-associated viruses, such as *BDP1, CDH23, COL11A2, LOXHD1, MET, MYO154, MYO3A, MYO7A, OTOG, OTOF, OTOGL, PCDH15, PTPRQ, STRC, TECTA, TARA,* etc. The length of their coding regions is more than 4 kb. Therefore, along with associated regulatory elements, the length will exceed the packaging limitation of the AAV vector. Although the problem is solved by using DNA recombinant dual vectors at present, the recombination efficiency of the DNA recombinant dual vectors packaged *in vivo* is low.

*OTOF* plays an important role in hearing in the deafness-related genes with coding sequences longer than 4 kb. OTOF protein is primarily expressed in the inner hair cells of the cochlea and has a major role in binding a calcium ion (Ca²⁺) to initiate the release of downstream neurotransmitters. Deletion or loss-of-function mutation of the *OTOF* gene causes deafness, DFNB9.

The *OTOF* gene (NCBI Gene ID: 9381) has different transcripts after transcription, including isoform 1 (NM_194248.3), isoform 2 (NM_004802.4), isoform 3 (NM_194322.3), isoform 4 (NM_194323.3), and isoform 5 (NM_001287489.2). They all originate from different cleavages of the same RNA, wherein variants associated with hearing in the inner ear hair cells are either isoform 1 or isoform 5. The length of the CDS regions of the two transcripts is 5,994 bp and the protein with a length of 1,997 amino acids is encoded.

For the congenital deafness caused by *OTOF* gene mutation, although AAV has the characteristics of non-integration delivery, long expression time, low immunogenicity, the packaging capacity of AAV is less than 4.7 kb. The problem of packaging limitation cannot meet the packaging of the *OTOF* gene (the total length of the *OTOF* gene and regulatory sequences exceeds 7 kb). To solve the packaging problem of the *OTOF,* there are three prevalent methods at present. Firstly, overload packaging is a choice. The method has been repoted to package a gene with a length of 7.5 kb into one AAV, the AAV is injected into the cochlea of mice, about 30% of the inner ear hair cells can express the OTOF protein within several weeks, and the mice hearing are recovered to about 58 dB. The overload packaging has low packaging efficiency, the product quality is not easy to control, and the transfection efficiency is low. Therefore, the overload packaging is not the best solution. Secondly, a coding sequence for a mini OTOF is used. OTOF is a C2 structural domain protein and consists of 6 C2 structural domains of A, B, C, D, E, and F and a TEM domain. The research shows that a mini OTOF consisting of a plurality of the structural domains can partially recover the function of the OTOF but cannot recover the hearing of animals. Thirdly, a dual-vector method is used with DNA recombination to produce a full-length mature OTOF mRNA for translation of the full length protein. Specifically, the dual-vector method can be divided into overlapping, trans-splicing or combination of the overlapping and trans-splicing.

Although the DNA recombination strategy can obtain a full-length functional OTOF protein (otoferlin), the recombination efficiency of the strategy is low. In order to overcome this problem, the protein recombination is probably the best choice. Especially, the protein trans-splicing mediated by intein is quick and high efficiency. Intein is firstly found in fungi. After then, many microorganisms such as bacteria, viruses and archaea are all found similar proteins. Intein has both intramolecular and intermolecular protein linkages. The intermolecular linkage of the intein may be naturally or artificially. An N-terminal and a C-terminal of the intein are respectively connected to two parts of a C-terminal and an N-terminal of a target protein. Therefore, a nucleophilic group of the first part can be attacked by an electrophilic group of the second part to form a covalent connection. The full length protein is formed through allosterism. In molecule design, it is only necessary that the first amino acid in the C-terminal part is serine, threonine or cysteine, etc.

Therefore, it's possible to improve the protein recombination efficiency in dual vector delivery considering the characteristics of protein recombination. The inventors of the present disclosure study and adopt the recombination strategy at a protein level and design the recombination of deafness-related proteins. The related method is to use the AAV vector to express the OTOF protein by an intein recombination method, thereby realizing function recovery and high-efficiency expression of abnormal genes.

### SUMMARY

Currently, the method utilizing the dual-vector AAV delivery and the DNA recombination has low recombination efficiency for OTOF expression. In addition, the current dual-vector delivery only realizes the effect of expression of OTOF protein in the unilateral cochlea and hearing recovery of the unilateral ear. If the hearing recovery of the bilateral ears is needed, the injection of the cochlea of the bilateral ears is inevitable. However, inconvenience and increased risk will be brought to patients due to the bilateral injection. In order to solve the above technical problems, the present disclosure is to provide a dual-vector system capable of expressing an OTOF protein and an adeno-associated virus packaged thereby, which can realize the hearing recover of bilateral ears through administration to the unilateral ear, being suitable for the field of double vector delivery in the gene therapy for deafness.

A first technical solution provided by the present disclosure is a dual-vector system for expressing an OTOF protein. The dual-vector system comprises two nucleotide sequences.

A first nucleotide sequence comprises two ITR sequences and an expression cassette inserted between the ITR sequences.

A second nucleotide sequence comprises two ITR sequences and an expression cassette inserted between the ITR sequences.

The expression cassette of the first nucleotide sequence comprises a promoter, an N-terminal coding sequence of OTOF, an N-terminal coding sequence of Intein, and a polyA.

The expression cassette of the second nucleotide sequence comprises a promoter, a C-terminal coding sequence of Intein, a C-terminal coding sequence of OTOF, and a polyA.

An OTOF amino acid sequence is shown in SEQ ID NO: 1 or SEQ ID NO: 2 of a sequence listing.

A cleavage site is arranged on the OTOF amino acid sequence, the N-terminal coding sequence of OTOF is a nucleotide coding sequence from an N-terminal of the OTOF amino acid sequence to the cleavage site, and the C-terminal coding sequence of OTOF is a nucleotide coding sequence from an amino acid next to the cleavage site to a C-terminal of the OTOF amino acid sequence.

Furthermore, the OTOF cleavage site comprises but is not limited to a preceding amino acid of a serine, a threonine or a cysteine in the OTOF amino acid sequence, wherein the sequence is from the N-terminal to the C-terminal of OTOF.

The promoter comprises but is not limited to a CAG promoter, a CMV promoter, a CBA promoter, a UbC promoter, an SFFV promoter, an EF1α promoter, a PGK promoter, or promoters for encoding genes *Myo7A, Myol5, Atohl, POU4F3, Lhx3, Myo6, α9AchR, α10AchR, OTOF,* etc.

The polyA is an adenine modification at a tail end of mRNA during a maturation process and can make the mRNA more stable, and the PolyA sequence in the present disclosure comprises AATAAA and variants thereof, comprising but not limited to ATTAAA, AGTAAA, CATAAA, TATAAA, GATAAA, ACTAAA, AATATA, AAGAAA, AATAAT, AAAAAA, AATGAA, AATCAA, AACAAA, AATCAA, AATAAC, AATAGA, AATTAA or AATAAG.

Furthermore, the ITR sequences (inverted terminal repeat sequence) are derived from AAV1, AAV2, AAV3, AAV4, AAV5, AAV6, AAV7, AAV8 or AAV9.

The expression cassette further comprises other expression elements, comprising but not limited to an expression regulatory element and a tag element.

Furthermore, the expression regulatory element comprises but is not limited to a regulatory element having the following functions: (1) the regulatory element is used for regulating the expression of a target protein, e.g., an IRES, for initiating translation of a downstream gene; (2) the regulatory element is used for expressing miRNA or siRNA; (3) the regulatory element is an intron; (4) the regulatory element is a localization sequence to localize the expression of the target protein to the nucleus, cytoplasm, various organelles, or outside the cell; (5) the regulatory element is a sequence accelerating protein degradation, e.g., a PEST sequence; (6) the regulatory element may also be a part of a kozak sequence which is GNCNCN, e.g. GCCACC; (7) the regulatory element is an enhancer which may be derived from an SV40 virus, a CMV virus or an adenovirus; and (8) the regulatory element may be a WPRE.

Furthermore, the tag element comprises but is not limited to, such as, FLAG, HA, MYC, a fluorescent protein, a luciferase, an SUMO protein, ubiquitin, GST, etc.

Furthermore, a nucleotide sequence of an *OTOF* coding gene is shown in SEQ ID NO: 3 or SEQ ID NO: 4 of a sequence listing.

Furthermore, the Intein comprises but is not limited to Intein sequences in proteins of MxeGyrA, pabPolIII, MjaKlbA, SspDnaB, SceVMA, SspDnaE, NpuDnaE, AvaDnaE, CraDnaE, CspDnaE, CwaDnaE, MchtDnaE, OliDnaE, TerDnaE, gp41-1, gp41-8, IMPDH-1, RmaDnaB, etc.

Furthermore, for the OTOF amino acid sequence shown in SEQ ID NO: 1 or 2, the cleavage site comprises but is not limited to 827th, 930th, 954th, and 1,130th amino acid residues.

More furthermore, for the OTOF amino acid sequence shown in SEQ ID NO: 1 or 2, the cleavage site is the 827^{th} amino acid residue, namely, a nucleotide coding sequence of a 1-827^{th} amino acid sequence is the N-terminal coding sequence of OTOF and a nucleotide coding sequence of a 828-1,997^{th} amino acid sequence is the C-terminal coding sequence of OTOF.

More furthermore, for the OTOF amino acid sequence shown in SEQ ID NO: 1 or 2, the cleavage site is the 930th amino acid residue, namely, a nucleotide coding sequence of a 1-930th amino acid sequence is the N-terminal coding sequence of OTOF and a nucleotide coding sequence of a 931-1,997th amino acid sequence is the C-terminal coding sequence of OTOF.

More furthermore, for the OTOF amino acid sequence shown in SEQ ID NO: 1 or 2, the cleavage site is the 954th amino acid residue, namely, a nucleotide coding sequence of a 1-954th amino acid sequence is the N-terminal coding sequence of OTOF and a nucleotide coding sequence of a 955-1,997th amino acid sequence is the C-terminal coding sequence of OTOF.

More furthermore, for the OTOF amino acid sequence shown in SEQ ID NO: 1 or 2, the cleavage site is the 1,130th amino acid residue, namely, a nucleotide coding sequence of a 1-1,130th amino acid sequence is the N-terminal coding sequence of OTOF and a nucleotide coding sequence of a 1,131-1,997th amino acid sequence is the C-terminal coding sequence of OTOF.

Preferably, the OTOF amino acid sequence is shown in SEQ ID NO: 2.

Preferably, the first nucleotide sequence is an expression cassette inserting the first nucleotide sequence in a plasmid comprising an ITR and between the ITR sequences; and the second nucleotide sequence is an expression cassette inserting the second nucleotide sequence in a plasmid comprising an ITR and between the ITR sequences.

Furthermore, the plasmid comprising an ITR comprises but is not limited to a pAAV, pAAV-CMV, pX601, pX551 and pAAV-MCS plasmid, etc.

A second technical solution provided by the present disclosure is a packaging vector system for an adeno-associated virus, wherein the packaging vector system comprises the dual-vector system for expressing an OTOF protein according to the first technical solution, a vector carrying AAV *rep* and *cap* genes, and a helper virus vector, and the vectors are packaged into AAV vectors.

Furthermore, the vector carrying AAV *rep* and *cap* genes comprises but is not limited to AAV1, AAV2, AAV5, AAV8, AAV9, Anc80, PHP.eB, AAV-DJ or AAVrh.10 vectors.

Furthermore, the helper virus vector is an adenovirus or herpesvirus helper virus vector, preferably a pHelper plasmid.

A third technical solution provided by the present disclosure is a packaging method for an adeno-associated virus, wherein the adeno-associated virus packaging vector system according to the second technical solution is transferred into a host cell for packaging.

Furthermore, dual vectors in the packaging vector system according to the second technical solution are respectively transferred into a host cell together with the vector carrying AAV *rep* and *cap* genes and the helper virus vector for packaging.

Furthermore, the host cell is a cell line capable of performing virus replication and with a stable inheritance, and comprises but is not limited to cells such as Hela-S3, HEK-293, HEK-293T, HEK-293FT, A549, Sf 9, etc.

Preferably, the host cell is HEK-293 or HEK-293Tcells.

A fourth technical solution provided by the present disclosure is an adeno-associated virus obtained by the packaging method according to the third technical solution, wherein the virus is a pair of viruses which are respectively packaged with an N-terminal coding sequence of OTOF and an N-terminal coding sequence of intein, and a C-terminal coding sequence of OTOF and a C-terminal coding sequence of Intein.

A fifth technical solution provided by the present disclosure is use of the dual-vector system according to the first technical solution or the adeno-associated virus according to the fourth technical solution, especially in preparing a drug or a preparation for treating a deafness disease or a hearing impairment or a hearing dysfunction.

Furthermore, the deafness disease or hearing impairment or hearing dysfunction is caused by a gene mutation including an *OTOF* gene and the mutation comprises but is not limited to a base substitution, a frameshift mutation, a deletion mutation, an insertion mutation, etc.

A sixth technical solution provided by the present disclosure is a preparation, or a formula or a drug prepared from the dual-vector system according to the first technical solution or the adeno-associated virus according to the fourth technical solution.

Furthermore, the preparation or formula or drug may be in any dosage form, comprising but not limited to injectable and ointment dosage forms.

Furthermore, in the preparation or formula or drug, the dual-vector system or adeno-associated virus is an only active component.

Furthermore, the preparation or formula or drug may comprise a common solvent and buffer, such as a common drug carrier and an adjuvant, comprising one or more of a neutral salt buffer, an acidic salt buffer, an alkaline salt buffer, glucose, mannose, mannitol, a protein, a polypeptide, an amino acid, an antibiotic, a chelating agent, an adjuvant or a preservative.

More furthermore, the buffer is a phosphate buffer, a Tris buffer, a 0.01% poloxamer PBS buffer or an HEPES buffer.

Furthermore, in the preparation or formula or drug, the active component may further be contained in other vectors, such as a nanoparticle, a liposome, a positive lipid particle, etc.

Furthermore, the preparation or formula or drug is administrated in a unilateral ear or bilateral ear manner.

Preferably, the preparation or formula or drug is administrated in a unilateral ear manner.

More furthermore, during the unilateral ear administration, the administration is cochlea injection and comprises but is not limited to injection through a round window, an oval window, a semicircular canal, and an alveus communis of the cochlea.

More furthermore, lifetime single administration or multiple administration is performed with a total dose of 1 × 10⁹-1 × 10¹³ virus genomes.

Beneficial effects are as follows:
The present disclosure applies the AAV dual vectors and Intein recombination method to the expression of *OTOF* and obviously improves the efficiency of a complete expression of an OTOF protein. Meanwhile, the unilateral cochlea administration achieves an effect of recovering bilateral hearing. After the unilateral ear administration, hearing of injected ears of *Otof-*/*-* mice can recover to a level of wild-type mice, while hearing of contralateral non-injected ears can also be improved to 60 decibels.

### BRIEF DESCRIPTION OF THE DRAWINGS

FIG. 1 is a pAAV-CMV plasmid map;
FIG. 2 is a pAAV-CMV-OTOF-N-S 1-Npu-N-intein plasmid map;
FIG. 3 is a pAAV-CMV-Npu-C-intein-OTOF-C-S1 plasmid map;
FIG. 4 is a pAAV-CMV-OTOF-N-S2-Npu-N-intein plasmid map;
FIG. 5 is a pAAV-CMV-Npu-C-intein-OTOF-C-S2 plasmid map;
FIG. 6 is a pAAV-CMV-OTOF-N-S4-Npu-N-intein plasmid map;
FIG. 7 is a pAAV-CMV-Npu-C-intein-OTOF-C-S4 plasmid map;
FIG. 8 is a pAAV-CMV-OTOF-N-S1-Rma-N-intein plasmid map;
FIG. 9 is a pAAV-CMV-Rma-C-intein-OTOF-C-S1 plasmid map;
FIG. 10 is a pAAV-CMV-OTOF-N-S2-Rma-N-intein plasmid map;
FIG. 11 is a pAAV-CMV-Rma-C-intein-OTOF-C-S2 plasmid map;
FIG. 12 is a pAAV-CMV-OTOF-N-S3-Rma-N-intein plasmid map;
FIG. 13 is a pAAV-CMV-Rma-C-intein-OTOF-C-S3 plasmid map;
FIG. 14 is a pAAV-CMV-OTOF-N-S4-Rma-N-intein plasmid map;
FIG. 15 is a pAAV-CMV-Rma-C-intein-OTOF-C-S4 plasmid map;
FIG. 16 is a pAAV-CMV-OTOF-FL plasmid map;
FIG. 17 is a pAAV-CMV-OTOF-N-AK plasmid map;
FIG. 18 is a pAAV-AK-OTOF-C-PolyA plasmid map;
FIG. 19 is a pAAV-CMV-OTOF-N-AP plasmid map;
FIG. 20 is a pAAV-AP-OTOF-C-PolyA plasmid map;
FIG. 21 is a pAAV-CMV-OTOF-N-TS plasmid map;
FIG. 22 is a pAAV-TS-OTOF-C-PolyA plasmid map;
FIG. 23 is an imaging result of example 6;
FIG. 24 is an imaging result of example 7;
FIG. 25 is an imaging result of example 8;
FIG. 26 is an imaging result of example 9;
FIG. 27 is an imaging result of example 10;
FIG. 28 is a comparison of results of OTOF Intein recombination and OTOF DNA recombination;
FIG. 29 is a comparison of bases of *Otof*^{*-*/*-*} gene mutant mice with wild-type mice;
FIG. 30 is a comparison of hearing of *Otof*^{*-*/*-*} gene mutant mice with wild-type mice;
FIG. 31 shows hearing recovery after 1 month in example 13;
FIG. 32 shows hearing recovery after 2 month in example 13;
FIG. 33 shows hearing recovery after 1 month in example 14;
FIG. 34 shows hearing recovery after 2 month in example 14;
FIG. 35 shows a hearing recovery effect of a mouse in a low-dose group;
FIG. 36 shows an expression of OTOF in wild-type and gene-deficient mice;
FIG. 37 shows expressions of OTOF in administered and contralateral ears of mice;
FIG. 38 shows statistics of an expression of OTOF in mice; and
FIG. 39 shows hearing of mice recovered by an AAV1 serotype OTOF.

### DESCRIPTION OF THE EMBODIMENTS

To make the objective, technical solutions and advantages of the present application clearer and more comprehensible, the present application will be further described below in detail in conjunction with specific examples. It should be understood that the specific examples described herein are merely intended to explain the patent, rather than to limit the present disclosure.

The present disclosure realizes a high-efficiency expression of an OTOF protein in a host by a method of combining dual vectors (or AAV dual vectors) delivery and intein recombination, further realizes the recovery of a deafness disease or hearing impairment or hearing dysfunction, and simultaneously achieves a technical effect of recovering hearing of bilateral ears by a unilateral ear administration.

The present disclosure utilizes an intein recombination method to express the OTOF protein. The related OTOF regions are: isoform 5 (NM_001287489.2) with a translated amino acid sequence of NP_001274418.1; and isoform 1 (NM_194248.3) with a translated amino acid sequence of NP_919224.1.

In some examples of the present disclosure, a target protein required for recovering or improving deafness is OTOF shown in SEQ ID NO: 1 or SEQ ID NO: 2. The target protein may further be a sequence with a similarity of 65%-100%, such as 99%, 98%, 97%, 96%, 95%, 90%, 85%, 80%, 75%, 70%, and 65% to any one of SEQ ID NO: 1 or 2. And, the protein may further have a suitable truncated protein, such as a protein with a length of 99%, 98%, 97%, 96%, 95%, 90%, 85%, 80%, 75%, 70%, 65%, and 60% of the full-length protein of any one of SEQ ID NO: 1 or 2. The protein may be appropriately inserted, such as a protein with a length of 101%, 102%, 103%, 104%, 105%, 110%, 115%, 120%, 125%, 130%, 135%, and 140% of a full-length protein. Related sequences have been listed in Table 1.

**Table 1 OTOF sequences and corresponding relationships**

| SEQ ID NO: | NCBI number | Feature |
|---|---|---|
| 3 | CCDS1725.1 | Isoform 1, nucleotide sequence |
| 1 | NP_919224.1 | Isoform 1, amino acid sequence |
| 4 | CCDS74497.1 | Isoform 5, nucleotide sequence |
| 2 | NP_001274418.1 | Isoform 5, amino acid sequence |

In addition to the nucleotide sequence of SEQ ID NO: 3 or 4 corresponds the amino acid sequence of SEQ ID NO: 1 or 2, the amino acid sequence may be codons optimized. The corresponding CAI may be 0.65-1.0, such as 1.0, 0.99, 0.98, 0.97, 0.96, 0.95, 0.90, 0.85, 0.80, 0.75, 0.70 or 0.65.

In addition to the OTOF protein sequences from human as described above, the OTOF protein sequences may also be selected from other animals, such as mice (protein sequences NP_001273350.1, NP_001300696.1, NP_001093865.1 or NP_114081.2), rats (protein sequence NP_001263649.1), pigs (protein sequence XP_020943388.1), monkeys (protein sequences XP_014967378.2, XP_014967379.2, XP_028687700.1, XP_014967380.2 or XP_028687701.1), and a sequence with a similarity of 65%-100%, such as 99%, 98%, 97%, 96%, 95%, 90%, 85%, 80%, 75%, 70%, and 65%.

Intein may splice a protein and function after or simultaneously with a protein translation to covalently link two different proteins. The earliest intein is found in fungi. It is predicted that the number of intein genes present in viruses, bacteria, archaea, and eukaryotic microorganisms is more than 600 by comparison and analysis of the sequences. Most inteins are complete proteins. In a small fraction of the inteins, an N-terminal and a C-terminal are separate, each linked to a portion of a protein, and subjected to recombination after the protein translation, and can form a complete protein through a nucleophilic chemical reaction and allosterism. In the present application, preferably, the N-terminal and the C-terminal of the intein are separated. The intein may be selected from inteins of MxeGyrA, pabPolIII, MjaKlbA, SspDnaB, SceVMA, SspDnaE, NpuDnaE, AvaDnaE, CraDnaE, CspDnaE, CwaDnaE, MchtDnaE, OliDnaE, TerDnaE, gp41-1, gp41-8, IMPDH-1, RmaDnaB, etc. Parts of intein amino acid sequences used in the examples of the present disclosure are shown in Table 2:

**Table 2 Part of intein amino acid sequences**

| Name | | Sequence |
|---|---|---|
| Npu Dna E | Npu Dna E-N | CLSYETEILTVEYGLLPIGKIVEKRIECTVYSVDNNGNIYTQPVAQWH DRGEQEVFEYCLEDGSLIRATKDHKFMTVDGQMLPIDEIFERELDLM RVDNLPN |
| | Npu Dna E-C | IKIATRKYLGKQNVYDIGVERDHNFALKNGFIASN |
| Rma Dna B | Rma Dna B-N | CLAGDTLITLADGRRVPIRELVSQQNFSVWALNPQTYRLERARVSRA FCTGIKPVYRLTTRLGRSIRATANHRFLTPQGWKRVDELQPGDYLAL PRRIPTAS |
| | Rma Dna B-C | AAACPELRQLAQSDVYWDPIVSIEPDGVEEVFDLTVPGPHNFVANDII AHN |

In the present disclosure, when the target protein is assembled into two vectors by using an intein recombination method. The OTOF is divided into an N-terminal and a C-terminal by taking a cleavage site as a boundary and the cleavage is performed as follows: 1) the OTOF can be packaged into an AAV and 2) a first amino acid at the C-terminal is serine, threonine or cysteine. The N-terminal of the intein is subjected to a fusion expression to the C-terminal of the N-terminal of the OTOF protein, and the C-terminal of the intein is subjected to a fusion expression to the N-terminal of the C-terminal of the OTOF protein.

Preferably, the OTOF protein is split into two segments with lengths of the N-terminal and the C-terminal approximately equivalent, or the length of the N-terminal/C-terminal of 0.3-3, such as the length of the N-terminal/C-terminal of 0.3, 0.4, 0.5, 0.6, 0.7, 0.8, 0.9, 1.0, 1.2, 1.5, 1.7, 1.8, 2.0, 2.0, 2.5, or 3.0.

More preferably, the splitting of the C-terminal of the N-terminal of the OTOF protein comprises but is not limited to cases listed in Table 3 of example 1.

More preferably, for the amino acid sequence of the OTOF protein shown in SEQ ID NO: 1 or 2, the N-terminal and the C-terminal are split into 1-827th amino acids and 828-1,997th amino acids, 1-930th amino acids and 931-1,997th amino acids, 1-954th amino acids and 955-1,997th amino acids, and 1-1,130th amino acids and 1,131-1,997th amino acids.

In the present disclosure, when the intein method is used to assemble the target protein into two vectors, that is, a dual-vector system for expressing an OTOF protein is constructed. The dual-vector system comprises two nucleotide sequences.

A first nucleotide sequence comprises two ITR sequences and an expression cassette inserted between the ITR sequences, and a second nucleotide sequence comprises two ITR sequences and an expression cassette inserted between the ITR sequences; the ITR is a sequence used for recognizing an adeno-associated protein and packaging DNA, and involved in recovery and replication of an adeno-associated virus genome; and preferably, the ITR sequence is derived from AAV1, AAV2, AAV3, AAV4, AAV5, AAV6, AAV7, AAV8, AAV9, and an ITR sequence having a similarity of 65%-100%, such as 99%, 98%, 97%, 96%, 95%, 90%, 85%, 80%, 75%, 70%, and 65% of the sequences described above.

The N-terminal of the intein is subjected to fusion expression to the C-terminal of the N-terminal of the OTOF protein and the obtained sequence is constructed into the expression cassette of the first nucleotide sequence, such that the expression cassette of the first nucleotide sequence comprises a promoter, an N-terminal coding sequence of OTOF, an N-terminal coding sequence of intein, and a polyA.

The C-terminal of the intein is subjected to fusion expression to the N-terminal of the C-terminal of the OTOF protein and the obtained sequence is constructed into the expression cassette of the second nucleotide sequence, such that the expression cassette of the second nucleotide sequence comprises a promoter, a C-terminal coding sequence of intein, a C-terminal coding sequence of OTOF, and a polyA.

The first nucleotide sequence and the second nucleotide sequence can both construct the N-terminal or C-terminal sequence of the OTOF in no particular order.

In the present disclosure, the above-mentioned promoter refers to a related sequence capable of promoting transcription of a downstream target protein, is generally used for recruiting transcription factors, is a sequence capable of expressing the target protein in a specific space and time, and comprises but is not limited to the following promoters: a promoter for a RNA polymerase II or a promoter for a RNA polymerase III. The promoter may also be classified as a broadly expressing promoter, such as a CMV promoter, a CAG promoter, etc. The promoter may also be a tissue specific promoter. Preferably, the promoter is highly expressed in an ear. Furthermore, the promoter is highly expressed at a cochlea or a vestibule. Furthermore, the promoter is highly expressed at the cochlea. The tissue specific promoter may be derived from a part or all of a sequence 1-10,000 bp upstream of a transcription initiation site of an *OTOF* gene and a sequence with a similarity of 65%-100%, such as 99%, 98%, 97%, 96%, 95%, 90%, 85%, 80%, 75%, 70%, and 65%. Preferably, the promoter suitable for the present disclosure comprises but is not limited to a CAG promoter, a CMV promoter, a CBA promoter, a UbC promoter, an SFFV promoter, an EF1α promoter, a PGK promoter, or promoters corresponding to genes *Myo7A, Myol5, Atohl, POU4F3, Lhx3, Myo6, α9AchR, α10AchR, OTOF,* etc.

PolyA refers to an adenine modification of a tail end during an mRNA maturation and can make the mRNA more stable. The PolyA sequence in the present disclosure comprises AATAAA and variants thereof, comprising ATTAAA, AGTAAA, CATAAA, TATAAA, GATAAA, ACTAAA, AATATA, AAGAAA, AATAAT, AAAAAA, AATGAA, AATCAA, AACAAA, AATCAA, AATAAC, AATAGA, AATTAA or AATAAG, etc.

The first or second nucleotide sequence may further comprise 1 or several regulatory elements, such as 2, 3, 4, 5, etc.; and the first and second regulatory elements may or may not be identical. The regulatory element comprises but is not limited to the following regulatory element: (1) the regulatory element is used for regulating an expression of a target protein, e.g., an IRES, for initiating translation of a downstream gene and the IRES is a sequence capable of initiating the protein translation within mRNA, such as the IRES can be derived from FMDV, EMCV, HRV, HIV, HAV, HCV, PV and other viruses; (2) the regulatory element is used for expressing miRNA and siRNA sequences; (3) the regulatory element is an intron; (4) the regulatory element is a localization sequence to localize the expression of the target protein to the nucleus, cytoplasm or various organelles, and secrete the target protein outside the cell; (5) the regulatory element is a sequence accelerating protein degradation, e.g., a PEST sequence; (6) the regulatory element may also be a part of a kozak sequence which is GNCNCN, e.g. GCCACC; (7) the regulatory element is an enhancer which refers to a sequence capable of enhancing gene expression, such as a CMV enhancer, an SV40 virus enhancer or an adenovirus enhancer, may be an upstream or a downstream of the target gene as well as within the gene, may or may not contain tissue specificity, and may or may not transcribe eRNA; and (8) the regulatory element may be a WPRE.

The first or second nucleotide sequence may further comprise 1 or several tags, such as 2, 3, 4, 5, etc.; and the first and second tags may or may not be identical. The tag may be, such as FLAG, HA, MYC, a fluorescent protein, a luciferase, etc., and may also be used to improve properties of a protein, such as an SUMO protein, ubiquitin, GST, etc.

The first or second nucleotide sequence constructed above may be a plasmid and a linear or circular nucleic acid in other forms, can co-express the N-terminal and C-terminal of the target protein to a cell, a tissue, an organ, and an individual, preferably a cochlea of the scope of the patent through proper selection of expression and regulatory elements, Preferably, the present disclosure uses an ITR-containing plasmid including pAAV, pAAV-CMV, pX601, pX551, pAAV-MCS, etc. to load a target gene expression cassette, followed by AAV packaging.

When the constructed first and second nucleotide sequences are virus vectors, the sequences may be used for AAV packaging in any forms. The first and second nucleotide sequences are respectively transferred into a host cell together with a vector carrying AAV *rep* and *cap* genes and a helper virus vector to be packaged to obtain an adeno-associated virus; and the host cell is a cell line capable of performing virus replication and with a stable inheritance, and comprises but is not limited to cells such as Hela-S3, HEK-293, HEK-293T, HEK-293FT, A549, Sf 9, etc.; and preferably, the host cell is HEK-293 or HEK-293Tcells. The packaged AAV sample may contain an empty virus with the content of 0%-99%, such as 0%, 5%, 10%, 15%, 20%, 25%, 30%, 35%, 40%, 45%, 50%, 55%, 60%, 65%, 70%, 75%, 80%, 85%, 90%, and 95%, preferably 0%-50%.

The virus obtained by the packaging method is a pair of adeno-associated viruses respectively packaged with an N-terminal coding sequence of OTOF and an N-terminal coding sequence of intein, and a C-terminal coding sequence of intein and a C-terminal coding sequence of OTOF. The pair of adeno-associated viruses may co-express the N-terminal and C-terminal of the target protein to a cell, a tissue, an organ, and an individual. Intein may splice a protein and function after or simultaneously with a protein translation to covalently link two different proteins to obtain a full-length functional OTOF protein. The adeno-associated virus is suitable for a variety of mammalian cells, such as human, murine, dog, pig, rabbit, hamster, sheep, cat, horse or non-human primate cells. The adeno-associated virus can be used in ear, inner ear, cochlear, and inner ear hair cells, or cells not related to the ear. The cells may be *in vitro, in vivo,* or *ex vivo.*

Therefore, the dual vectors or adeno-associated virus may be used in preparation of a drug for a deafness disease or hearing impairment or hearing dysfunction. The deafness disease or hearing impairment or hearing dysfunction is caused by a gene mutation including an *OTOF* gene and the mutation comprises but is not limited to a base substitution, a frameshift mutation, a deletion mutation, an insertion mutation, etc.

A preparation or formula prepared from the dual vectors or adeno-associated virus may be a powder or a solution. The preparation or formula may comprise a common solvent and buffer, such as a common drug carrier and an adjuvant, comprising a neutral salt buffer, an acidic salt buffer, an alkaline salt buffer, glucose, mannose, mannitol, a protein, a polypeptide, an amino acid, an antibiotic, a chelating agent, an adjuvant or a preservative. The buffer comprises a phosphate buffer, a Tris buffer, and an HEPES buffer. The preparation or formula may further be contained in other vectors, such as a nanoparticle, a liposome, a positive lipid particle, etc. The solution may simulate components of a perilymph: NaCl at a concentration of 20-200 mM, KCl at a concentration of 1-5 mM, CaCl₂ at a concentration of 0.1-10 mM, glucose at a concentration of 1-10 mM and HEPES at a concentration of 2-50 mM, with a pH of 6-9. The solution is finally sterile, can be dissolved in water, and can also be glycerol, ethanol, polyalcohol, oil, etc. The solution may be in a form ready for use or may be diluted for use.

The present disclosure uses the following administration modes: in the present disclosure, the adeno-associated virus obtained from the dual vectors or final packaging may be delivered to an ear by various methods, and may be delivered to the ear from areas of a round window, an oval window, a semicircular canal, an alveus communis, etc. with or without the use of auxiliary tools. The administration is one-time administration or multiple administrations according to protein expression and hearing recovery, the delivery dose may be 1-200 µL of AAV containing 1×10⁹-1×10¹³ virus genomes, for example, delivering 1-2 µL of AAV containing 1×10⁹, 1×10¹⁰ or 1×10¹¹ virus genomes in a mouse and delivering 10-100 µL of viruses containing 1×10⁹-1×10¹³ virus genomes in human. The adeno-associated virus may be delivered in a unilateral ear or bilateral ear manner. The unilateral ear delivery is preferred in the present disclosure to finally realize an effect of recovering bilateral hearing.

The present disclosure will be further described below in conjunction with specific examples.

### Example 1 Selection of intein cleavage site of OTOF (SEQ ID NO: 1 or 2)

A cleavage site was arranged on an OTOF amino acid sequence, an N-terminal coding sequence of OTOF was a nucleotide coding sequence from an N-terminal of the OTOF amino acid sequence to the cleavage site, and a C-terminal coding sequence of OTOF was a nucleotide coding sequence from an amino acid next to the cleavage site to a C-terminal of the OTOF amino acid sequence. The N-terminal of the OTOF was connected and fused with an N-terminal of intein and a C-terminal of the intein was connected and fused with the C-terminal of the OTOF. There were many choices for cleavage sites of the OTOF, some of which were listed in Table 3.

**Table 3 Part of cleavage sites applicable to OTOF shown in SEQ ID NO: 1 or 2**

| | | | | | | |
|---|---|---|---|---|---|---|
| Amino acid 1-10-Intein N-terminal, Intein C-terminal amino acid 11-1,997; | Amino acid 1-28-Intein N-terminal, Intein C-terminal amino acid 29-1,997; | Amino acid 1-31-Intein N-terminal, Intein C-terminal amino acid 32-1,997; | Amino acid 1-53-Intein N-terminal, Intein C-terminal amino acid 54-1,997; | Amino acid 1-54-Intein N-terminal, Intein C-terminal amino acid 55-1,997; | Amino acid 1-69-Intein N-terminal, Intein C-terminal amino acid 70-1,997; | Amino acid 1-73-Intein N-terminal, Intein C-terminal amino acid 74-1,997; |
| Amino acid 1-159-Intein N-terminal, Intein C-terminal amino acid 160-1,997; | Amino acid 1-166-Intein N-terminal, Intein C-terminal amino acid 167-1,997; | Amino acid 1-173-Intein N-terminal, Intein C-terminal amino acid 174-1,997; | Amino acid 1-176-Intein N-terminal, Intein C-terminal amino acid 177-1,997; | Amino acid 1-185-Intein N-terminal, Intein C-terminal amino acid 186-1,997; | Amino acid 1-219-Intein N-terminal, Intein C-terminal amino acid 220-1,997; | Amino acid 1-221-Intein N-terminal, Intein C-terminal amino acid 222-1,997; |
| Amino acid 1-307-Intein N-terminal, Intein C-terminal amino acid 308-1,997; | Amino acid 1-319-Intein N-terminal, Intein C-terminal amino acid 320-1,997; | Amino acid 1-323-Intein N-terminal, Intein C-terminal amino acid 324-1,997; | Amino acid 1-329-Intein N-terminal, Intein C-terminal amino acid 330-1,997; | Amino acid 1-335-Intein N-terminal, Intein C-terminal amino acid 336-1,997; | Amino acid 1-345-Intein N-terminal, Intein C-terminal amino acid 346-1,997; | Amino acid 1-359-Intein N-terminal, Intein C-terminal amino acid 360-1,997; |
| Amino acid 1-520-Intein N-terminal, Intein C-terminal amino acid 521-1,997; | Amino acid 1-541-Intein N-terminal, Intein C-terminal amino acid 542-1,997; | Amino acid 1-563-Intein N-terminal, Intein C-terminal amino acid 564-1,997; | Amino acid 1-580-Intein N-terminal, Intein C-terminal amino acid 581-1,997; | Amino acid 1-586-Intein N-terminal, Intein C-terminal amino acid 587-1,997; | Amino acid 1-587-Intein N-terminal, Intein C-terminal amino acid 588-1,997; | Amino acid 1-599-Intein N-terminal, Intein C-terminal amino acid 600-1,997; |
| Amino acid 1-715-Intein N-terminal, Intein C-terminal amino acid 716-1,997; | Amino acid 1-753-Intein N-terminal, Intein C-terminal amino acid 754-1,997; | Amino acid 1-767-Intein N-terminal, Intein C-terminal amino acid 768-1,997; | Amino acid 1-775-Intein N-terminal, Intein C-terminal amino acid 776-1,997; | Amino acid 1-784-Intein N-terminal, Intein C-terminal amino acid 785-1,997; | Amino acid 1-785-Intein N-terminal, Intein C-terminal amino acid 786-1,997; | Amino acid 1-796-Intein N-terminal, Intein C-terminal amino acid 797-1,997; |
| Amino acid 1-970-Intein N-terminal, Intein C-terminal amino acid 971-1,997; | Amino acid 1-976-Intein N-terminal, Intein C-terminal amino acid 977-1,997; | Amino acid 1-977-Intein N-terminal, Intein C-terminal amino acid 978-1,997; | Amino acid 1-980-Intein N-terminal, Intein C-terminal amino acid 981-1,997; | Amino acid 1-992-Intein N-terminal, Intein C-terminal amino acid 993-1,997; | Amino acid 1-1,040-Intein N-terminal, Intein C-terminal amino acid 1,041-1,997; | Amino acid 1-1,130-Intein N-terminal, Intein C-terminal amino acid 1,131-1,997; |
| Amino acid 1-1,258-Intein N-terminal, Intein C-terminal amino acid 1,259-1,997; | Amino acid 1-1,259-Intein N-terminal, Intein C-terminal amino acid 1,260-1,997; | Amino acid 1-1,260-Intein N-terminal, Intein C-terminal amino acid 1,261-1,997; | Amino acid 1-1,262-Intein N-terminal, Intein C-terminal amino acid 1,263-1,997; | Amino acid 1-1,289-Intein N-terminal, Intein C-terminal amino acid 1,290-1,997; | Amino acid 1-1,323-Intein N-terminal, Intein C-terminal amino acid 1,324-1,997; | Amino acid 1-1,329-Intein N-terminal, Intein C-terminal amino acid 1,330-1,997; |
| Amino acid 1-1,416-Intein N-terminal, Intein C-terminal amino acid 1,417-1,997; | Amino acid 1-1,442-Intein N-terminal, Intein C-terminal amino acid 1,443-1,997; | Amino acid 1-1,455-Intein N-terminal, Intein C-terminal amino acid 1,456-1,997; | Amino acid 1-1,468-Intein N-terminal, Intein C-terminal amino acid 1,469-1,997; | Amino acid 1-1,475-Intein N-terminal, Intein C-terminal amino acid 1,476-1,997; | Amino acid 1-1,485-Intein N-terminal, Intein C-terminal amino acid 1,486-1,997; | Amino acid 1-1,533-Intein N-terminal, Intein C-terminal amino acid 1,534-1,997; |
| Amino acid 1-1,724-Intein N-terminal, | Amino acid 1-1,756-Intein N-terminal, | Amino acid 1-1,757-Intein N-terminal, | Amino acid 1-1,781-Intein N-terminal, | Amino acid 1-1,808-Intein N-terminal, | Amino acid 1-1,812-Intein N-terminal, | Amino acid 1-1,815-Intein N-terminal, |
| Intein C-terminal amino acid 1,725-1,997; | Intein C-terminal amino acid 1,757-1,997; | Intein C-terminal amino acid 1,758-1,997; | Intein C-terminal amino acid 1,782-1,997; | Intein C-terminal amino acid 1,809-1,997; | Intein C-terminal amino acid 1,813-1,997; | Intein C-terminal amino acid 1,816-1,997; |
| Amino acid 1-37-Intein N-terminal, Intein C-terminal amino acid 38-1,997; | Amino acid 1-112-Intein N-terminal, Intein C-terminal amino acid 113-1,997; | Amino acid 1-275-Intein N-terminal, Intein C-terminal amino acid 276-1,997; | Amino acid 1-293-Intein N-terminal, Intein C-terminal amino acid 294-1,997; | Amino acid 1-374-Intein N-terminal, Intein C-terminal amino acid 375-1,997; | Amino acid 1-491-Intein N-terminal, Intein C-terminal amino acid 492-1,997; | Amino acid 1-602-Intein N-terminal, Intein C-terminal amino acid 603-1,997; |
| Amino acid 1-926-Intein N-terminal, Intein C-terminal amino acid 927-1,997; | Amino acid 1-930-Intein N-terminal, Intein C-terminal amino acid 931-1,997; | Amino acid 1-994-Intein N-terminal, Intein C-terminal amino acid 995-1,997; | Amino acid 1-1,003-Intein N-terminal, Intein C-terminal amino acid 1,004-1,997; | Amino acid 1-1,064-Intein N-terminal, Intein C-terminal amino acid 1,065-1,997; | Amino acid 1-1,161-Intein N-terminal, Intein C-terminal amino acid 1,162-1,997; | Amino acid 1-1,208-Intein N-terminal, Intein C-terminal amino acid 1,209-1,997; |
| Amino acid 1-1,958-Intein N-terminal, Intein C-terminal amino acid 1,959-1,997; | Amino acid 1-1,838-Intein N-terminal, Intein C-terminal amino acid 1,839-1,997; | Amino acid 1-1,878-Intein N-terminal, Intein C-terminal amino acid 1,879-1,997; | Amino acid 1-1,952-Intein N-terminal, Intein C-terminal amino acid 1,953-1,997; | Amino acid 1-1,984-Intein N-terminal, Intein C-terminal amino acid 1,985-1,997; | Amino acid 1-1,869-Intein N-terminal, Intein C-terminal amino acid 1,870-1,997; | Amino acid 1-1,903-Intein N-terminal, Intein C-terminal amino acid 1,904-1,997; |
| Amino acid 1-8-Intein N-terminal, Intein C-terminal amino acid 9-1,997; | Amino acid 1-23-Intein N-terminal, Intein C-terminal amino acid 24-1,997; | Amino acid 1-46-Intein N-terminal, Intein C-terminal amino acid 47-1,997; | Amino acid 1-79-Intein N-terminal, Intein C-terminal amino acid 80-1,997; | Amino acid 1-96-Intein N-terminal, Intein C-terminal amino acid 97-1,997; | Amino acid 1-98-Intein N-terminal, Intein C-terminal amino acid 99-1,997; | Amino acid 1-109-Intein N-terminal, Intein C-terminal amino acid 110-1,997; |
| Amino acid 1-285-Intein N-terminal, Intein C-terminal amino acid 286-1,997; | Amino acid 1-291-Intein N-terminal, Intein C-terminal amino acid 292-1,997; | Amino acid 1-331-Intein N-terminal, Intein C-terminal amino acid 332-1,997; | Amino acid 1-342-Intein N-terminal, Intein C-terminal amino acid 343-1,997; | Amino acid 1-387-Intein N-terminal, Intein C-terminal amino acid 388-1,997; | Amino acid 1-394-Intein N-terminal, Intein C-terminal amino acid 395-1,997; | Amino acid 1-433-Intein N-terminal, Intein C-terminal amino acid 434-1,997; |
| Amino acid 1-585-Intein N-terminal, Intein C-terminal amino acid 586-1,997; | Amino acid 1-588-Intein N-terminal, Intein C-terminal amino acid 589-1,997; | Amino acid 1-596-Intein N-terminal, Intein C-terminal amino acid 597-1,997; | Amino acid 1-631-Intein N-terminal, Intein C-terminal amino acid 632-1,997; | Amino acid 1-635-Intein N-terminal, Intein C-terminal amino acid 636-1,997; | Amino acid 1-687-Intein N-terminal, Intein C-terminal amino acid 688-1,997; | Amino acid 1-695-Intein N-terminal, Intein C-terminal amino acid 696-1,997; |
| Amino acid 1-995-Intein N-terminal, Intein C-terminal amino acid 996-1,997; | Amino acid 1-1,001-Intein N-terminal, Intein C-terminal amino acid 1,002-1,997; | Amino acid 1-1,005-Intein N-terminal, Intein C-terminal amino acid 1,006-1,997; | Amino acid 1-1,050-Intein N-terminal, Intein C-terminal amino acid 1,051-1,997; | Amino acid 1-1,083-Intein N-terminal, Intein C-terminal amino acid 1,084-1,997; | Amino acid 1-1,182-Intein N-terminal, Intein C-terminal amino acid 1,183-1,997; | Amino acid 1-1,215-Intein N-terminal, Intein C-terminal amino acid 1,216-1,997; |
| Amino acid 1-1,337-Intein N-terminal, Intein C-terminal amino acid 1,338-1,997; | Amino acid 1-1,361-Intein N-terminal, Intein C-terminal amino acid 1,362-1,997; | Amino acid 1-1,385-Intein N-terminal, Intein C-terminal amino acid 1,386-1,997; | Amino acid 1-1,427-Intein N-terminal, Intein C-terminal amino acid 1,428-1,997; | Amino acid 1-1,435-Intein N-terminal, Intein C-terminal amino acid 1,436-1,997; | Amino acid 1-1,443-Intein N-terminal, Intein C-terminal amino acid 1,444-1,997; | Amino acid 1-1,476-Intein N-terminal, Intein C-terminal amino acid 1,477-1,997; |
| Amino acid 1-1,599-Intein N-terminal, Intein C- | Amino acid 1-1,616-Intein N-terminal, Intein C- | Amino acid 1-1,643-Intein N-terminal, Intein C- | Amino acid 1-1,658-Intein N-terminal, Intein C- | Amino acid 1-1,687-Intein N-terminal, Intein C- | Amino acid 1-1,719-Intein N-terminal, Intein C- | Amino acid 1-1,740-Intein N-terminal, Intein C- |
| terminal amino acid 1,600-1,997; | terminal amino acid 1,617-1,997; | terminal amino acid 1,644-1,997; | terminal amino acid 1,659-1,997; | terminal amino acid 1,688-1,997; | terminal amino acid 1,720-1,997; | terminal amino acid 1,741-1,997; |
| Amino acid 1-91-Intein N-terminal, Intein C-terminal amino acid 92-1,997; | Amino acid 1-110-Intein N-terminal, Intein C-terminal amino acid 111-1,997; | Amino acid 1-126-Intein N-terminal, Intein C-terminal amino acid 127-1,997; | Amino acid 1-137-Intein N-terminal, Intein C-terminal amino acid 138-1,997; | Amino acid 1-145-Intein N-terminal, Intein C-terminal amino acid 146-1,997; | Amino acid 1-155-Intein N-terminal, Intein C-terminal amino acid 156-1,997; | Amino acid 1-158-Intein N-terminal, Intein C-terminal amino acid 159-1,997; |
| Amino acid 1-224-Intein N-terminal, Intein C-terminal amino acid 225-1,997; | Amino acid 1-233-Intein N-terminal, Intein C-terminal amino acid 234-1,997; | Amino acid 1-237-Intein N-terminal, Intein C-terminal amino acid 238-1,997; | Amino acid 1-246-Intein N-terminal, Intein C-terminal amino acid 247-1,997; | Amino acid 1-256-Intein N-terminal, Intein C-terminal amino acid 257-1,997; | Amino acid 1-286-Intein N-terminal, Intein C-terminal amino acid 287-1,997; | Amino acid 1-290-Intein N-terminal, Intein C-terminal amino acid 291-1,997; |
| Amino acid 1-365-Intein N-terminal, Intein C-terminal amino acid 366-1,997; | Amino acid 1-366-Intein N-terminal, Intein C-terminal amino acid 367-1,997; | Amino acid 1-434-Intein N-terminal, Intein C-terminal amino acid 435-1,997; | Amino acid 1-467-Intein N-terminal, Intein C-terminal amino acid 468-1,997; | Amino acid 1-471-Intein N-terminal, Intein C-terminal amino acid 472-1,997; | Amino acid 1-472-Intein N-terminal, Intein C-terminal amino acid 473-1,997; | Amino acid 1-501-Intein N-terminal, Intein C-terminal amino acid 502-1,997; |
| Amino acid 1-601-Intein N-terminal, Intein C-terminal amino acid 602-1,997; | Amino acid 1-619-Intein N-terminal, Intein C-terminal amino acid 620-1,997; | Amino acid 1-647-Intein N-terminal, Intein C-terminal amino acid 648-1,997; | Amino acid 1-671-Intein N-terminal, Intein C-terminal amino acid 672-1,997; | Amino acid 1-683-Intein N-terminal, Intein C-terminal amino acid 684-1,997; | Amino acid 1-685-Intein N-terminal, Intein C-terminal amino acid 686-1,997; | Amino acid 1-686-Intein N-terminal, Intein C-terminal amino acid 687-1,997; |
| Amino acid 1-844-Intein N-terminal, Intein C-terminal amino acid 845-1,997; | Amino acid 1-854-Intein N-terminal, Intein C-terminal amino acid 855-1,997; | Amino acid 1-866-Intein N-terminal, Intein C-terminal amino acid 867-1,997; | Amino acid 1-872-Intein N-terminal, Intein C-terminal amino acid 873-1,997; | Amino acid 1-900-Intein N-terminal, Intein C-terminal amino acid 901-1,997; | Amino acid 1-918-Intein N-terminal, Intein C-terminal amino acid 919-1,997; | Amino acid 1-950-Intein N-terminal, Intein C-terminal amino acid 951-1,997; |
| Amino acid 1-1,168-Intein N-terminal, Intein C-terminal amino acid 1,169-1,997; | Amino acid 1-1,169-Intein N-terminal, Intein C-terminal amino acid 1,170-1,997; | Amino acid 1-1,219-Intein N-terminal, Intein C-terminal amino acid 1,220-1,997; | Amino acid 1-1,223-Intein N-terminal, Intein C-terminal amino acid 1,224-1,997; | Amino acid 1-1,224-Intein N-terminal, Intein C-terminal amino acid 1,225-1,997; | Amino acid 1-1,236-Intein N-terminal, Intein C-terminal amino acid 1,237-1,997; | Amino acid 1-1,239-Intein N-terminal, Intein C-terminal amino acid 1,240-1,997; |
| Amino acid 1-1,334-Intein N-terminal, Intein C-terminal amino acid 1,335-1,997; | Amino acid 1-1,348-Intein N-terminal, Intein C-terminal amino acid 1,349-1,997; | Amino acid 1-1,367-Intein N-terminal, Intein C-terminal amino acid 1,368-1,997; | Amino acid 1-1,387-Intein N-terminal, Intein C-terminal amino acid 1,388-1,997; | Amino acid 1-1,388-Intein N-terminal, Intein C-terminal amino acid 1,389-1,997; | Amino acid 1-1,390-Intein N-terminal, Intein C-terminal amino acid 1,391-1,997; | Amino acid 1-1,394-Intein N-terminal, Intein C-terminal amino acid 1,395-1,997; |
| Amino acid 1-1,543-Intein N-terminal, Intein C-terminal amino acid 1,544-1,997; | Amino acid 1-1,549-Intein N-terminal, Intein C-terminal amino acid 1,550-1,997; | Amino acid 1-1,554-Intein N-terminal, Intein C-terminal amino acid 1,555-1,997; | Amino acid 1-1,585-Intein N-terminal, Intein C-terminal amino acid 1,586-1,997; | Amino acid 1-1,598-Intein N-terminal, Intein C-terminal amino acid 1,599-1,997; | Amino acid 1-1,612-Intein N-terminal, Intein C-terminal amino acid 1,613-1,997; | Amino acid 1-1,646-Intein N-terminal, Intein C-terminal amino acid 1,647-1,997; |
| Amino acid 1-710-Intein N-terminal, Intein C-terminal amino acid 711-1,997; | Amino acid 1-768-Intein N-terminal, Intein C-terminal amino acid 769-1,997; | Amino acid 1-770-Intein N-terminal, Intein C-terminal amino acid 771-1,997; | Amino acid 1-771-Intein N-terminal, Intein C-terminal amino acid 772-1,997; | Amino acid 1-797-Intein N-terminal, Intein C-terminal amino acid 798-1,997; | Amino acid 1-827-Intein N-terminal, Intein C-terminal amino acid 828-1,997; | Amino acid 1-882-Intein N-terminal, Intein C-terminal amino acid 883-1,997; |
| Amino acid 1-1,250-Intein N-terminal, Intein C-terminal amino acid 1,251-1,997; | Amino acid 1-1,254-Intein N-terminal, Intein C-terminal amino acid 1,255-1,997; | Amino acid 1-1,457-Intein N-terminal, Intein C-terminal amino acid 1,458-1,997; | Amino acid 1-1,591-Intein N-terminal, Intein C-terminal amino acid 1,592-1,997; | Amino acid 1-1,619-Intein N-terminal, Intein C-terminal amino acid 1,620-1,997; | Amino acid 1-1,678-Intein N-terminal, Intein C-terminal amino acid 1,679-1,997; | Amino acid 1-1,863-Intein N-terminal, Intein C-terminal amino acid 1,864-1,997; |
| Amino acid 1-120-Intein N-terminal, Intein C-terminal amino acid 121-1,997; | Amino acid 1-123-Intein N-terminal, Intein C-terminal amino acid 124-1,997; | Amino acid 1-148-Intein N-terminal, Intein C-terminal amino acid 149-1,997; | Amino acid 1-226-Intein N-terminal, Intein C-terminal amino acid 227-1,997; | Amino acid 1-229-Intein N-terminal, Intein C-terminal amino acid 230-1,997; | Amino acid 1-230-Intein N-terminal, Intein C-terminal amino acid 231-1,997; | Amino acid 1-258-Intein N-terminal, Intein C-terminal amino acid 259-1,997; |
| Amino acid 1-466-Intein N-terminal, Intein C-terminal amino acid 467-1,997; | Amino acid 1-484-Intein N-terminal, Intein C-terminal amino acid 485-1,997; | Amino acid 1-511-Intein N-terminal, Intein C-terminal amino acid 512-1,997; | Amino acid 1-530-Intein N-terminal, Intein C-terminal amino acid 531-1,997; | Amino acid 1-542-Intein N-terminal, Intein C-terminal amino acid 543-1,997; | Amino acid 1-546-Intein N-terminal, Intein C-terminal amino acid 547-1,997; | Amino acid 1-579-Intein N-terminal, Intein C-terminal amino acid 580-1,997; |
| Amino acid 1-750-Intein N-terminal, Intein C-terminal amino acid 751-1,997; | Amino acid 1-787-Intein N-terminal, Intein C-terminal amino acid 788-1,997; | Amino acid 1-819-Intein N-terminal, Intein C-terminal amino acid 820-1,997; | Amino acid 1-878-Intein N-terminal, Intein C-terminal amino acid 879-1,997; | Amino acid 1-887-Intein N-terminal, Intein C-terminal amino acid 888-1,997; | Amino acid 1-904-Intein N-terminal, Intein C-terminal amino acid 905-1,997; | Amino acid 1-954-Intein N-terminal, Intein C-terminal amino acid 955-1,997; |
| Amino acid 1-1,242-Intein N-terminal, Intein C-terminal amino acid 1,243-1,997; | Amino acid 1-1,243-Intein N-terminal, Intein C-terminal amino acid 1,244-1,997; | Amino acid 1-1,263-Intein N-terminal, Intein C-terminal amino acid 1,264-1,997; | Amino acid 1-1,269-Intein N-terminal, Intein C-terminal amino acid 1,270-1,997; | Amino acid 1-1,281-Intein N-terminal, Intein C-terminal amino acid 1,282-1,997; | Amino acid 1-1,288-Intein N-terminal, Intein C-terminal amino acid 1,289-1,997; | Amino acid 1-1,311-Intein N-terminal, Intein C-terminal amino acid 1,312-1,997; |
| Amino acid 1-1,501-Intein N-terminal, Intein C-terminal amino acid 1,502-1,997; | Amino acid 1-1,523-Intein N-terminal, Intein C-terminal amino acid 1,524-1,997; | Amino acid 1-1,557-Intein N-terminal, Intein C-terminal amino acid 1,558-1,997; | Amino acid 1-1,568-Intein N-terminal, Intein C-terminal amino acid 1,569-1,997; | Amino acid 1-1,575-Intein N-terminal, Intein C-terminal amino acid 1,576-1,997; | Amino acid 1-1,590-Intein N-terminal, Intein C-terminal amino acid 1,591-1,997; | Amino acid 1-1,596-Intein N-terminal, Intein C-terminal amino acid 1,597-1,997; |
| Amino acid 1-1,752-Intein N-terminal, Intein C-terminal amino acid 1,753-1,997; | Amino acid 1-1,775-Intein N-terminal, Intein C-terminal amino acid 1,776-1,997; | Amino acid 1-1,783-Intein N-terminal, Intein C-terminal amino acid 1,784-1,997; | Amino acid 1-1,819-Intein N-terminal, Intein C-terminal amino acid 1,820-1,997; | Amino acid 1-1,828-Intein N-terminal, Intein C-terminal amino acid 1,829-1,997; | Amino acid 1-1,859-Intein N-terminal, Intein C-terminal amino acid 1,860-1,997; | Amino acid 1-1,864-Intein N-terminal, Intein C-terminal amino acid 1,865-1,997; |
| Amino acid 1-1,914-Intein N-terminal, Intein C-terminal amino acid 1,915-1,997; | Amino acid 1-1,941-Intein N-terminal, Intein C-terminal amino acid 1,942-1,997; | Amino acid 1-1,959-Intein N-terminal, Intein C-terminal amino acid 1,960-1,997; | | | | |

### Example 2 Construction of first/second nucleotide sequence by using pAAV-CMV plasmid containing ITR sequence

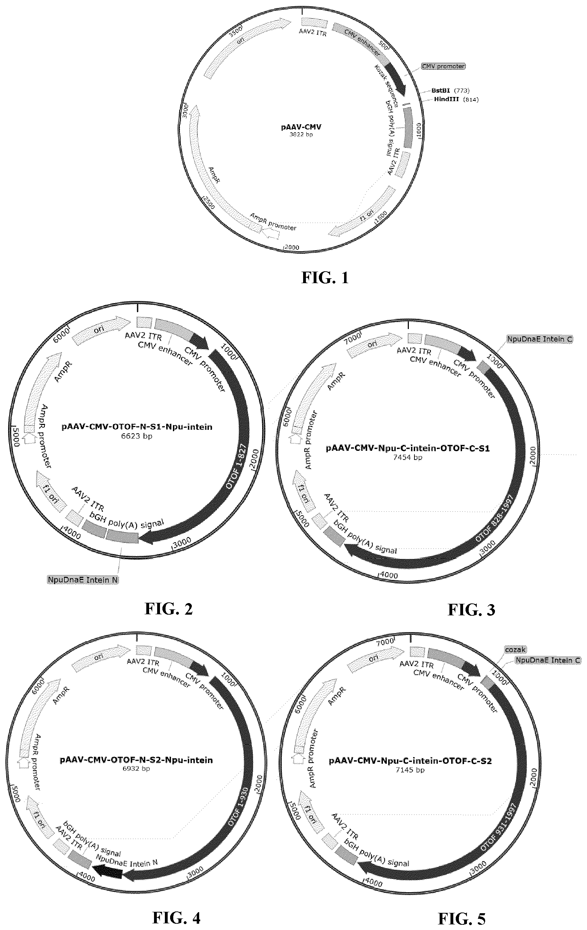

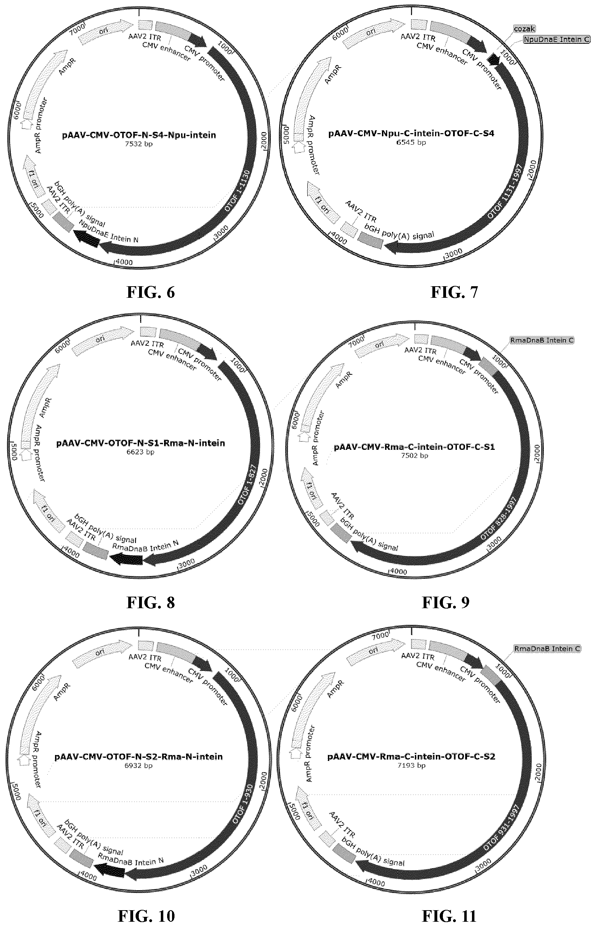

In an amino acid sequence of an OTOF protein shown in SEQ ID NO: 2, a 827th amino acid residue was used as a cleavage site (S1), a 930th amino acid residue was used as a cleavage site (S2), and a 1,130th amino acid residue was used as a cleavage site (S4) respectively; and NpuDnaE was used as intein and a pAAV-CMV plasmid (FIG. 1 and a sequence shown as SEQ ID NO: 9) as a vector to construct a first/second nucleotide sequence, namely a dual vector system for expressing the OTOF protein, which specifically comprises the following steps:
the pAAV-CMV plasmid was digested with BstBI and HindIII, and a plasmid fragment was recovered from the gel. A gene synthesis method was utilized to synthesize a gene fragment 1 (an encoding gene of 1-827th amino acids of OTOF and an N-terminal of NpuDnaE intein), a gene fragment 2 (an encoding gene of a C-terminal of NpuDnaE intein and a 828-1,997th amino acids of OTOF), a gene fragment 3 (an encoding gene of 1-930th amino acids of OTOF and the N-terminal of NpuDnaE intein), a gene fragment 4 (an encoding gene of the C-terminal of NpuDnaE intein and 931-1,997th amino acids of OTOF), a gene fragment 5 (an encoding gene of 1-1,130th amino acids of OTOF and an N-terminal of NpuDnaE intein), and a gene fragment 6 (an encoding gene of the C-terminal of NpuDnaE intein and 1,131-1,997th amino acids of OTOF), and the gene fragments were digested by BstBI and HindIII. The digested pAAV-CMV plasmid was respectively ligated with the digested gene fragments 1-6 by using a T4 ligase system. After the ligation, 2 µL of the ligation product was added to 50 µL of DH5 α competent cells, the cells were ice-bathed for 30 min, heat-shocked for 1 min, and immediately placed on ice for 1 min, 200 µL of an LB liquid medium was added, the mixture was incubated at 37°C for 30 min, and 200 µL of the bacterial solution was evenly applied to an ampicillin-resistant solid LB medium and incubated overnight at 37°C. 5 monoclones were picked and cultured in a shaker, and sequenced correctly. The following 6 plasmids were obtained for subsequent examples:
pAAV-CMV-OTOF-N-S1-Npu-N-intein plasmid (plasmid map shown in FIG. 2);
pAAV-CMV-Npu-C-intein-OTOF-C-S1 plasmid (plasmid map shown in FIG. 3);
pAAV-CMV-OTOF-N-S2-Npu-N-intein plasmid (plasmid map shown in FIG. 4);
pAAV-CMV-Npu-C-intein-OTOF-C-S2 plasmid (plasmid map shown in FIG. 5);
pAAV-CMV-OTOF-N-S4-Npu-N-intein plasmid (plasmid map shown in FIG. 6); and
pAAV-CMV-Npu-C-intein-OTOF-C-S4 plasmid (plasmid map shown in FIG. 7).
pAAV-CMV-OTOF-N-S1-Npu-N-intein and pAAV-CMV-Npu-C-intein-OTOF-C-S1;
pAAV-CMV-OTOF-N-S2-Npu-N-intein and pAAV-CMV-Npu-C-intein-OTOF-C-S2; pAAV-CMV-OTOF-N-S4-Npu-N-intein and pAAV-CMV-Npu-C-intein-OTOF-C-S4; and the above plasmid transcription products all contained covalently linked OTOF (a part) and NpuDnaE intein (a part). Transcription products were listed in Table 4.

**Table 4 Transcription and translation products of different N-terminals and C-terminals of Npu fused OTOF**

| No. | Name of plasmid | Transcription and translation product |
|---|---|---|
| 1 | pAAV-CMV-OTOF-N-S1-Npu-N-intein | OTOF 1-827-NpuDnaE intein N-terminal |
| 2 | pAAV-CMV-Npu-C-intein-OTOF-C-S1 | NpuDnaE intein C terminal-OTOF 828-1,997 |
| 3 | pAAV-CMV-OTOF-N-S2-Npu-N-intein | OTOF 1-930-NpuDnaE intein N-terminal |
| 4 | pAAV-CMV-Npu-C-intein-OTOF-C-S2 | NpuDnaE intein C terminal-OTOF 931-1,997 |
| 5 | pAAV-CMV-OTOF-N-S4-Npu-N-intein | OTOF 1-1130-NpuDnaE intein N-terminal |
| 6 | pAAV-CMV-Npu-C-intein-OTOF-C-S4 | NpuDnaE intein C terminal-OTOF 1131-1,997 |

### Example 3 Construction of first/second nucleotide sequence by using pAAV-CMV plasmid containing ITR sequence

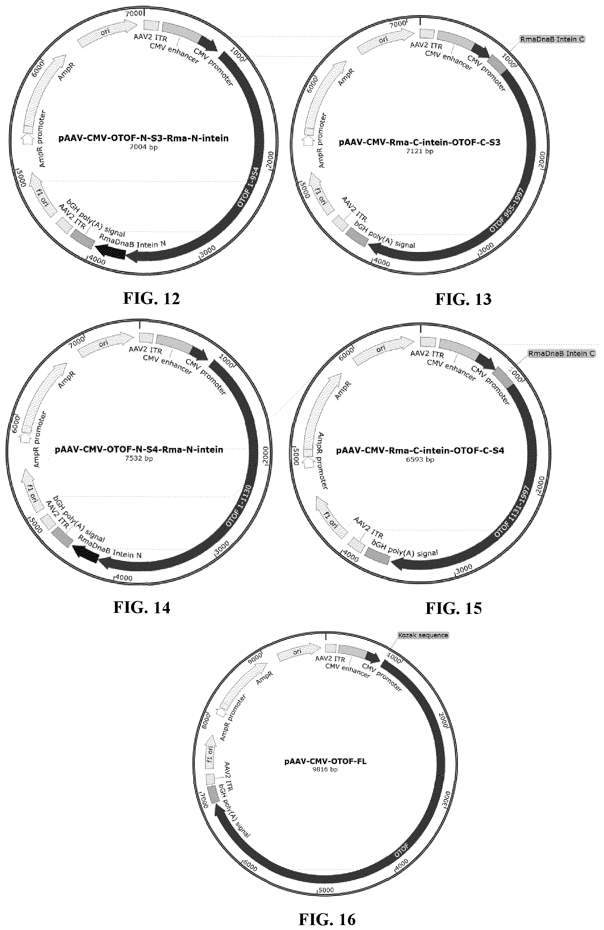

In an amino acid sequence of an OTOF protein shown in SEQ ID NO: 2, a 827th amino acid residue was used as a cleavage site (S1), a 930th amino acid residue was used as a cleavage site (S2), a 954th amino acid residue was used as a cleavage site (S3), and a 1,130th amino acid residue was used as a cleavage site (S4) respectively; and RmaDnaB intein was used and a pAAV-CMV plasmid (FIG. 1 and a sequence shown as SEQ ID NO: 9) was used as a vector to construct a first/second nucleotide sequence, namely a dual-vector system for expressing the OTOF protein, which specifically comprises the following steps:
the pAAV-CMV plasmid was digested with BstBI and HindIII, and a plasmid fragment was recovered from the gel. A gene synthesis method was utilized to synthesize a gene fragment 7 (an encoding gene of 1-827th amino acids of OTOF and an N-terminal of RmaDnaB intein as shown in SEQ ID NO: 5 (further comprising a part of sequence connected with the plasmid)), a gene fragment 8 (an encoding gene of a C-terminal of RmaDnaB intein and a 828-1,997th amino acids of OTOF as shown in SEQ ID NO: 6 (further comprising a part of sequence connected with the plasmid)), a gene fragment 9 (an encoding gene of 1-930th amino acids of OTOF and the N-terminal of RmaDnaB intein as shown in SEQ ID NO: 7 (further comprising a part of sequence connected with the plasmid)), a gene fragment 10 (an encoding gene of the C-terminal of RmaDnaB intein and 931-1,997th amino acids of OTOF as shown in SEQ ID NO: 8 (further comprising a part of sequence connected with the plasmid)), a gene fragment 11 (an encoding gene of 1-954th amino acids of OTOF and the N-terminal of RmaDnaB intein), a gene fragment 12 (an encoding gene of the C-terminal of RmaDnaB intein and 955-1,997th amino acids of OTOF), a gene fragment 13 (an encoding gene of 1-1,130th amino acids of OTOF and the N-terminal of RmaDnaB intein), and a gene fragment 14 (an encoding gene of the C-terminal of RmaDnaB intein and 1,131-1,997th amino acids of OTOF), and the gene fragments were digested by BstBI and HindIII. The digested pAAV-CMV plasmid was respectively ligated with the digested gene fragments 7-14 by using a T4 ligase system. After the ligation, 2 µL of the ligation product was added to 50 µL of DH5α competent cells, the cells were ice-bathed for 30 min, heat-shocked for 1 min, and immediately placed on ice for 1 min, 200 µL of an LB liquid medium was added, the mixture was incubated at 37°C for 30 min, and 200 µL of the bacterial solution was evenly applied to an ampicillin-resistant solid LB medium and incubated overnight at 37°C. 5 monoclones were picked and cultured in a shaker, and sequenced correctly. The following 8 plasmids were obtained for subsequent examples:
pAAV-CMV-OTOF-N-S1-Rma-N-intein plasmid (plasmid map shown in FIG. 8);
pAAV-CMV-Rma-C-intein-OTOF-C-S1 plasmid (plasmid map shown in FIG. 9);
pAAV-CMV-OTOF-N-S2-Rma-N-intein plasmid (plasmid map shown in FIG. 10);
pAAV-CMV-Rma-C-intein-OTOF-C-S2 plasmid (plasmid map shown in FIG. 11);
pAAV-CMV-OTOF-N-S3-Rma-N-intein plasmid (plasmid map shown in FIG 12);
pAAV-CMV-Rma-C-intein-OTOF-C-S3 plasmid (plasmid map shown in FIG 13);
pAAV-CMV-OTOF-N-S4-Rma-N-intein plasmid (plasmid map shown in FIG. 14); and
pAAV-CMV-Rma-C-intein-OTOF-C-S4 plasmid (plasmid map shown in FIG. 15).
pAAV-CMV-OTOF-N-S1-Rma-N-intein and pAAV-CMV-Rma-C-intein-OTOF-C-S1; pAAV-CMV-OTOF-N-S2-Rma-N-intein and pAAV-CMV-Rma-C-intein-OTOF-C-S2;
pAAV-CMV-OTOF-N-S3-Rma-N-intein and pAAV-CMV-Rma-C-intein-OTOF-C-S3;
pAAV-CMV-OTOF-N-S4-Rma-N-intein and pAAV-CMV-Rma-C-intein-OTOF-C-S4; and the above plasmid transcription products all contained covalently linked OTOF (a part) and RmaDnaB intein (a part). Transcription products were listed in Table 5.

**Table 5 Transcription and translation products of different N-terminals and C-terminals of Rma fused OTOF**

| No. | Name of plasmid | Transcription and translation product |
|---|---|---|
| 1 | pAAV-CMV-OTOF-N-S1-Rma-N-intein | OTOF 1-827- RmaDnaB intein N-terminal |
| 2 | pAAV-CMV-Rma-C-intein-OTOF-C-S1 | RmaDnaB intein C terminal-OTOF 828-1,997 |
| 3 | pAAV-CMV-OTOF-N-S2-Rma-N-intein | OTOF 1-930- RmaDnaB intein N-terminal |
| 4 | pAAV-CMV-Rma-C-intein-OTOF-C-S2 | **RmaDnaB intein** C terminal-OTOF 931-1,997 |
| 5 | pAAV-CMV-OTOF-N-S3-Rma-N-intein | OTOF 1-954-RmaDnaB intein N-terminal |
| 6 | pAAV-CMV-Rma-C-intein-OTOF-C-S3 | RmaDnaB intein C terminal-OTOF 955-1,997 |
| 7 | pAAV-CMV-OTOF-N-S4-Rma-N-intein | OTOF 1-1130- RmaDnaB intein N-terminal |
| 8 | pAAV-CMV-Rma-C-intein-OTOF-C-S4 | RmaDnaB intein C terminal-OTOF 1,131-1,997 |

### Example 4 Construction of different recombinant plasmids by OTOF shown in SEQ ID NO: 2

According to the method similar to example 2 and example 3, a full-length plasmid capable of expressing an OTOF protein was constructed: a pAAV-CMV-OTOF-FL plasmid (including a CMV promoter, *OTOF* isoform 5 (SEQ ID NO: 4), and bGH PolyA, and a map is shown in FIG. 16).

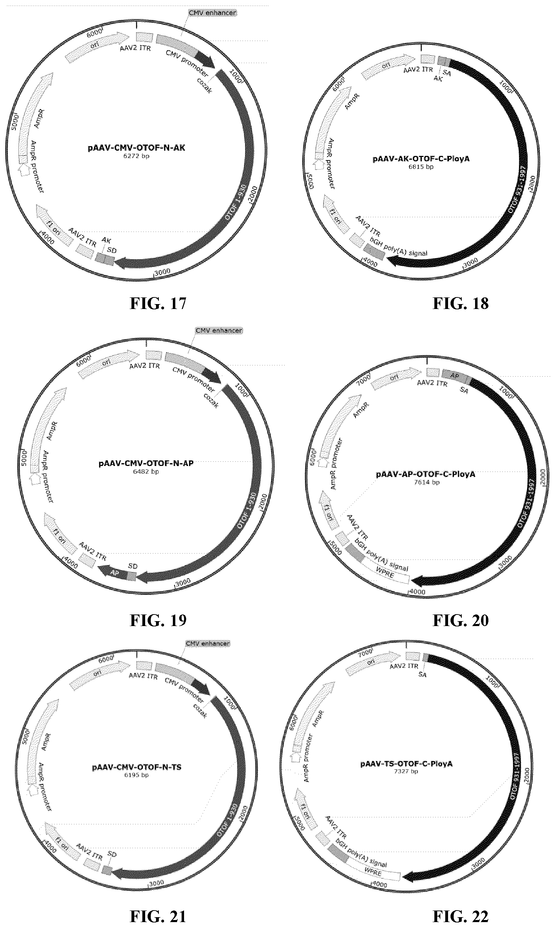

According to the method similar to example 2 and example 3, a plasmid for DNA recombination was constructed:
a pAAV-CMV-OTOF-N-AK plasmid (including a CMV promoter, a 1-930th amino acid coding sequence of OTOF, an SD sequence, and an AK sequence, and a map is shown in FIG. 17) and a pAAV-AK-OTOF-C-PloyA plasmid (including an AK sequence, an SA sequence, a 931-1,997th amino acid coding sequence of OTOF, and a bGH polyA sequence, and a map is shown in FIG. 18);
a pAAV-CMV-OTOF-N-AP plasmid (including a CMV promoter, a 1-930th amino acid coding sequence of OTOF, an SD sequence, and an AK sequence, and a map is shown in FIG. 19) and a pAAV-AP-OTOF-C-PloyA plasmid (including an AP sequence, an SA sequence, a 931-1,997th amino acid coding sequence of OTOF, and a bGH polyA sequence, and a map is shown in FIG. 20); and
a pAAV-CMV-OTOF-N-TS plasmid (including a CMV promoter, a 1-930th amino acid coding sequence of OTOF, and an SD sequence, and a map is shown in FIG. 21) and a pAAV-TS-OTOF-C-PloyA plasmid (including an SA sequence, a 931-1,997th amino acid coding sequence of OTOF, and a bGH polyA sequence, and a map is shown in FIG. 22).

AK and AP are sequences for DNA recombination after adeno-associated viruses after entering cells. After the DNA recombination, trans-splicing was performed through an SA-SD sequence, a complete mRNA was formed, and a full-length functional OTOF protein was expressed. pAAV-CMV-OTOF-N-TS and pAAV-TS-OTOF-C-PolyA adeno-associated viruses were recombined through ITR, trans-splicing was performed through an SA-SD sequence, a complete mature mRNA was formed, and a full-length functional OTOF protein was expressed.

### Example 5 Preparation of adeno-associated viruses

8 plasmids constructed by the method of the present disclosure in example 3 and 6 plasmids constructed in example 4 respectively along with a pHelper plasmid and a pRC plasmid of PHP.eB at a molar ratio of 1:1:1 were co-transfected into HEK-293T cells with a PEI transfection reagent (1 µg of the plasmids were added into about every million cells), the cells were cultured by using a DMEM medium containing 10% fetal bovine serum at a 5% carbon dioxide incubator at 37°C for 3 days, the cells were washed once with a PBS buffer, the cells were collected, and repeatedly frozen and thawed for five times, a solid NaCl was added to make a final concentration 500 mM, the cells were centrifuged at 10,000 g for half an hour, the obtained supernatant was taken and filtered with a 0.45-µm filter membrane, the filtrate was purified by using an iodixanol method, and a part of the sample was taken and concentrated to obtain an adeno-associated virus with a virus titer of 1±0.2×10¹³ virus genomes/ml, a solvent of a 0.01% poloxamer PBS buffer, and an empty shell rate of about 50%. Specific experimental systems were shown in Table 6 below. The viruses will be named according to the names of the plasmids later, for example, number 1 is an adenovirus packaged by pAAV-CMV-OTOF-N-S1-Rma-N-intein, a pHelper plasmid, and a PHP.eB and named a pAAV-CMV-OTOF-N-S1-Rma-N-intein adeno-associated virus.

A preparation method of the 0.01% poloxamer PBS buffer was as follows: (1) a PBS buffer was prepared: 137 mM of NaCl, 2.7 mM of KCl, 10 mM of Na₂HPO₄, 2 mM of KH₂PO₄ and the balance water; and (2) according to a mass-to-volume ratio, poloxamer F68 was added to the PBS buffer prepared in step (1) to make a final concentration of 0.01%.

The 0.01% poloxamer PBS buffer related in the present disclosure was all prepared by using the method.

**Table 6 Transfection system**

| Number | GOI plasmid | Helper virus vector | Vector carrying AAV *rep* and *cap* genes |
|---|---|---|---|
| 1 | pAAV-CMV-OTOF-N-S1-Rma-N-intein | pHelper plasmid | PHP.eB |
| 2 | pAAV-CMV-Rma-C-intein-OTOF-C-S1 | pHelper plasmid | PHP.eB |
| 3 | pAAV-CMV-OTOF-N-S2-Rma-N-intein | pHelper plasmid | PHP.eB |
| 4 | pAAV-CMV-Rma-C-intein-OTOF-C-S2 | pHelper plasmid | PHP.eB |
| 5 | pAAV-CMV-OTOF-N-S3-Rma-N-intein | pHelper plasmid | PHP.eB |
| 6 | pAAV-CMV-Rma-C-intein-OTOF-C-S3 | pHelper plasmid | PHP.eB |
| 7 | pAAV-CMV-OTOF-N-S4-Rma-N-intein | pHelper plasmid | PHP.eB |
| 8 | pAAV-CMV-Rma-C-intein-OTOF-C-S4 | pHelper plasmid | PHP.eB |
| 9 | pAAV-CMV-OTOF-N-AK | pHelper plasmid | PHP.eB |
| 10 | pAAV-AK-OTOF-C-PloyA | pHelper plasmid | PHP.eB |
| 11 | pAAV-CMV-OTOF-N-AP | pHelper plasmid | PHP.eB |
| 12 | pAAV-AP-OTOF-C-PloyA | pHelper plasmid | PHP.eB |
| 13 | pAAV-CMV-OTOF-N-TS | pHelper plasmid | PHP.eB |
| 14 | pAAV-TS-OTOF-C-PolyA | pHelper plasmid | PHP.eB |

### Example 6 Recombination of plasmids constructed in examples 2 and 3 in cells (at a plasmid ratio of 1:1)

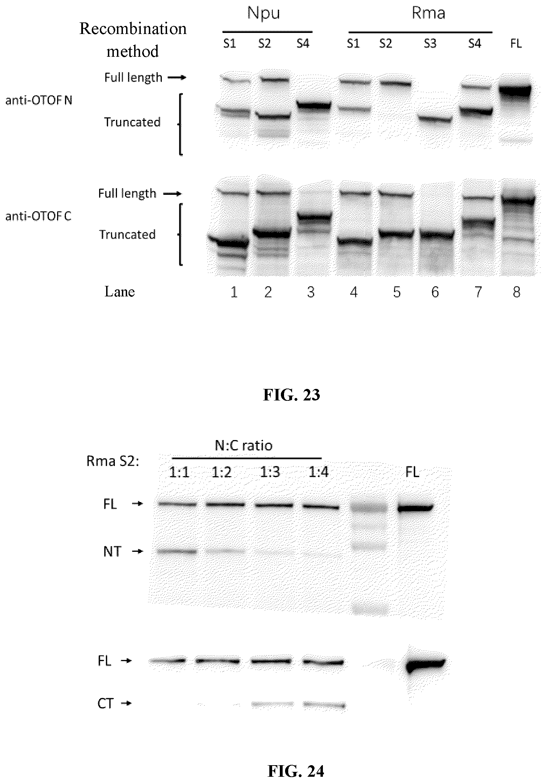

7 pairs of plasmids constructed in examples 2 and 3 and pAAV-CMV-OTOF-FL constructed in example 4 were respectively transfected into HEK-293T cells cultured in a six-well plate (the number of cells per well was about 1×10⁶, 2 µg of the plasmids were added into 100 µL of Opti-MEM to be mixed to obtain a plasmid premix, 4 µL of PEI was added into 100 µL of Opti-MEM to be mixed to obtain a PEI premix, the plasmid premix and the PEI premix were mixed, and the obtained mixture was put still for 10 min and dropped onto the cultured HEK-293T cells), the cells were cultured for 48 h, a cell culture medium was absorbed, 200 µL of a cell lysis buffer (1% triton-X 100, 50 mM of Tris-Hcl, and 1 mM of PMSF, pH = 7.4) was added, the cells were placed on ice for 10 min, a cell lysate was collected and centrifuged at 12,000 g and 4°C for 10 min, and a supernatant was kept. 1/4 volume of a 5×loading buffer (0.25 M of Tris HCl, 10% SDS, 0.05% bromophenol blue, 50% glycerin, and 0.25 M DTT) was added into the supernatant and the mixture was heated at 90°C for 10 min to obtain a protein sample. 50 µL of the protein sample was subjected to a polyacrylamide gel electrophoresis. After the protein was transferred from gel to a PVDF membrane, aiming at an OTOF N-terminal antibody (article No. A20266, abclonal) or a C-terminal antibody (article No. PA5-52935, Invitrogen), the protein was incubated at a normal temperature for 2 h and incubated with the corresponding HRP coupled secondary antibody for 1 h. An ECL reagent was added for imaging. The results were shown in FIG. 23. It can be seen that NpuS1, NpuS2, RmaS1, RmaS2, and RmaS4 all had relatively high efficiency of recombination. The NpuS2 and RmaS2 had the highest efficiency.

The amount of transfection plasmids was summarized in Table 7.

**Table 7 Amount of plasmid transfection**

| Lane | Name of recombinant plasmid | Amount |
|---|---|---|
| 1 (NpuS1) | pAAV-CMV-OTOF-N-S1-Npu-N-intein | 1 µg |
| | pAAV-CMV-Npu-C-intein-OTOF-C-S1 | 1 µg |
| 2 (NpuS2) | pAAV-CMV-OTOF-N-S2-Npu-N-intein | 1 µg |
| | pAAV-CMV-Npu-C-intein-OTOF-C-S2 | 1 µg |
| 3 (NpuS4) | pAAV-CMV-OTOF-N-S4-Npu-N-intein | 1 µg |
| | pAAV-CMV-Npu-C-intein-OTOF-C-S4 | 1 µg |
| 4 (RmaS1) | pAAV-CMV-OTOF-N-S1-Rma-N-intein | 1 µg |
| | pAAV-CMV-Rma-C-intein-OTOF-C-S1 | 1 µg |
| 5 (RmaS2) | pAAV-CMV-OTOF-N-S2-Rma-N-intein | 1 µg |
| | pAAV-CMV-Rma-C-intein-OTOF-C-S2 | 1 µg |
| 6 (RmaS3) | pAAV-CMV-OTOF-N-S3-Rma-N-intein | 1 µg |
| | pAAV-CMV-Rma-C-intein-OTOF-C-S3 | 1 µg |
| 7 (RmaS4) | pAAV-CMV-OTOF-N-S4-Rma-N-intein | 1 µg |
| | pAAV-CMV-Rma-C-intein-OTOF-C-S4 | 1 µg |
| 8 (FL) | pAAV-CMV-OTOF-FL | 2 µg |

### Example 7 Recombination of S2 plasmids at ratio of 1:2, etc. in cells

A pAAV-CMV-OTOF-N-S2-Rma-N-intein plasmid and a pAAV-CMV-Rma-C-intein-OTOF-C-S2 plasmid respectively at a mass ratio of 1:1 (1 µg, 1 µg), 1:2 (0.65 µg, 1.35 µg), 1:3 (0.5 µg, 1.5 µg), and 1:4 (0.4 µg, 1.6 µg), and 2 µg of pAAV-CMV-OTOF-FL were transfected into HEK-293T cells cultured in a six-well plate (the number of cells per well was about 1×10⁶, 2 µg of the plasmids were added into 100 µL of Opti-MEM to be mixed to obtain a plasmid premix, 4 µL of PEI was added into 100 µL of Opti-MEM to be mixed to obtain a PEI premix, the plasmid premix and the PEI premix were mixed, and the obtained mixture was put still for 10 min and dropped onto the cultured HEK-293T cells), the cells were cultured for 48 h, a cell culture medium was absorbed, 200 µL of a cell lysis buffer (1% triton-X 100, 50 mM of Tris-Hcl, and 1 mM of PMSF, pH = 7.4) was added, the cells were placed on ice for 10 min, a cell lysate was collected and centrifuged at 12,000 g and 4°C for 10 min, and a supernatant was kept. 1/4 volume of 5 ×loading buffer (0.25 M of Tris HCl, 10% SDS, 0.05% bromophenol blue, 50% glycerin, and 0.25 M DTT) was added into the supernatant and the mixture was heated at 90°C for 10 min to obtain a protein sample. 50 µL of the protein sample was subjected to a polyacrylamide gel electrophoresis. After the protein was transferred from gel to a PVDF membrane, aiming at an OTOF N-terminal antibody (A20266, abclonal) or a C-terminal antibody (PA5-52935, Invitrogen), the protein was incubated at a normal temperature for 2 h and incubated with the corresponding HRP coupled secondary antibody for 1 h. An ECL reagent was added for imaging. The results were shown in FIG. 24. It can be seen that RmaS2 had the highest transfection efficiency when N:C=1:2 during transfection.

### Example 8 Recombination of S2 plasmids at different ratios of 1:2.5, etc. in cells

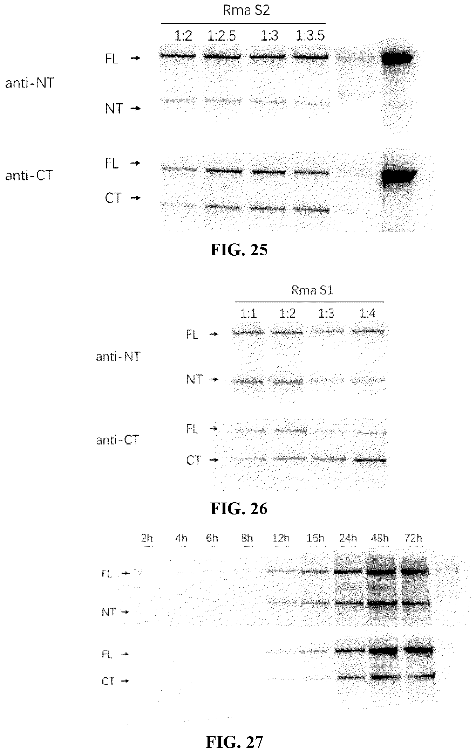

A pAAV-CMV-OTOF-N-S2-Rma-N-intein plasmid and a pAAV-CMV-Rma-C-intein-OTOF-C-S2 plasmid respectively at a mass ratio of 1:2 (0.65 µg, 1.35 µg), 1:2.5 (0.55 µg, 1.45 µg), 1:3 (0.5 µg, 1.5 µg), and 1:3.5 (0.4 µg, 1.6 µg), and 2 µg of pAAV-CMV-OTOF-FL were transfected into HEK-293T cells cultured in a six-well plate (the number of cells per well was about 1×10⁶, 2 µg of the plasmids were added into 100 µL of Opti-MEM to be mixed to obtain a plasmid premix, 4 µL of PEI was added into 100 µL of Opti-MEM to be mixed to obtain a PEI premix, the plasmid premix and the PEI premix were mixed, and the obtained mixture was put still for 10 min and dropped onto the cultured HEK-293T cells), the cells were cultured for 48 h, a cell culture medium was absorbed, 200 µL of a cell lysis buffer (1% triton-X 100, 50 mM of Tris-Hcl, and 1 mM of PMSF, pH = 7.4) was added, the cells were placed on ice for 10 min, a cell lysate was collected and centrifuged at 12,000 g and 4 °C for 10 min, and a supernatant was kept. 1/4 volume of a 5×loading buffer (0.25 M of Tris HCl, 10% SDS, 0.05% bromophenol blue, 50% glycerin, and 0.25 M DTT) was added into the supernatant and the mixture was heated at 90°C for 10 min to obtain a protein sample. 50 µL of the protein sample was subjected to a polyacrylamide gel electrophoresis. After the protein was transferred from gel to a PVDF membrane, aiming at an OTOF N-terminal antibody (A20266, abclonal) or a C-terminal antibody (PA5-52935, Invitrogen), the protein was incubated at a normal temperature for 2 h and incubated with the corresponding HRP coupled secondary antibody for 1 h. An ECL reagent was added for imaging. The results were shown in FIG. 25. It can be seen when a ratio of N:C was further adjusted to 1:2.5, the RmaS2 had the highest transfection efficiency.

### Example 9 Recombination of S1 plasmids at ratio of 1:2, etc. in cells

A pAAV-CMV-OTOF-N-S1-Rma-N-intein plasmid and a pAAV-CMV-Rma-C-intein-OTOF-C-S1 plasmid respectively at a mass ratio of 1:1 (1 µg, 1 µg), 1:2 (0.65 µg, 1.35 µg), 1:3 (0.5 µg, 1.5 µg), and 1:4 (0.4 µg, 1.6 µg), and 2 µg of pAAV-CMV-OTOF-FL were transfected into HEK-293T cells cultured in a six-well plate (the number of cells per well was about 1×10⁶, the N-terminal plasmid and C-terminal plasmid at the ratio were added into 100 µL of Opti-MEM to be mixed to obtain a plasmid premix, 4 µL of PEI was added into 100 µL of Opti-MEM to be mixed to obtain a PEI premix, the plasmid premix and the PEI premix were mixed, and the obtained mixture was put still for 10 min and dropped onto the cultured HEK-293T cells), the cells were cultured for 48 h, a cell culture medium was absorbed, 200 µL of a cell lysis buffer (1% triton-X 100, 50 mM of Tris-Hcl, and 1 mM of PMSF, pH = 7.4) was added, the cells were placed on ice for 10 min, a cell lysate was collected and centrifuged at 12,000 g and 4°C for 10 min, and a supernatant was kept. 1/4 volume of a 5 ×loading buffer (0.25 M of Tris HCl, 10% SDS, 0.05% bromophenol blue, 50% glycerin, and 0.25 M DTT) was added into the supernatant and the mixture was heated at 90°C for 10 min to obtain a protein sample. 50 µL of the protein sample was subjected to a polyacrylamide gel electrophoresis. After the protein was transferred from gel to a PVDF membrane, aiming at an OTOF N-terminal antibody (A20266, abclonal) or a C-terminal antibody (PA5-52935, Invitrogen), the protein was incubated at a normal temperature for 2 h and incubated with the corresponding HRP coupled secondary antibody for 1 h. An ECL reagent was added for imaging. The results were shown in FIG. 26. It can be seen that RmaS1 had the highest transfection efficiency when N:C=1:2 by optimizing a transfection ratio of the N-terminal and the C-terminal.

### Example 10 Transfection efficiency of different culture time

A pAAV-CMV-OTOF-N-S2-Rma-N-intein plasmid and a pAAV-CMV-Rma-C-intein-OTOF-C-S2 plasmid respectively at a mass ratio of 1:2.5 (0.55 µg, 1.45 µg) were transfected into HEK-293T cells cultured in a six-well plate (the number of cells per well was about 1×10⁶, 2 µg of the plasmids were added into 100 µL of Opti-MEM to be mixed to obtain a plasmid premix, 4 µL of PEI was added into 100 µL of Opti-MEM to be mixed to obtain a PEI premix, the plasmid premix and the PEI premix were mixed, and the obtained mixture was put still for 10 min and dropped onto the cultured HEK-293T cells), the cells were cultured for 2, 4, 6, 8, 12, 24, 48 and 72 h respectively, a cell culture medium was absorbed, 200 µL of a cell lysis buffer (1% triton-X 100, 50 mM of Tris-Hcl, and 1 mM of PMSF, pH = 7.4) was added, the cells were placed on ice for 10 min, a cell lysate was collected and centrifuged at 12,000 g and 4°C for 10 min, and a supernatant was kept. 1/4 volume of 5 × loading buffer (0.25 M of Tris HCl, 10% SDS, 0.05% bromophenol blue, 50% glycerin, and 0.25 M DTT) was added into the supernatant and the mixture was heated at 90°C for 10 min to obtain a protein sample. 50 µL of the protein sample was subjected to a polyacrylamide gel electrophoresis. After the protein was transferred from gel to a PVDF membrane, aiming at an OTOF N-terminal antibody (A20266, abclonal) or a C-terminal antibody (PA5-52935, Invitrogen), the protein was incubated at a normal temperature for 2 h and incubated with the corresponding HRP coupled secondary antibody for 1 h. An ECL reagent was added for imaging. The results were as shown in FIG. 27. It can be seen that OTOF recombined quickly by using the intein method. A ratio of an OTOF fragment and a full-length OTOF was basically the same at different time points.

### Example 11 Comparison of OTOF intein recombination and OTOF DNA recombination

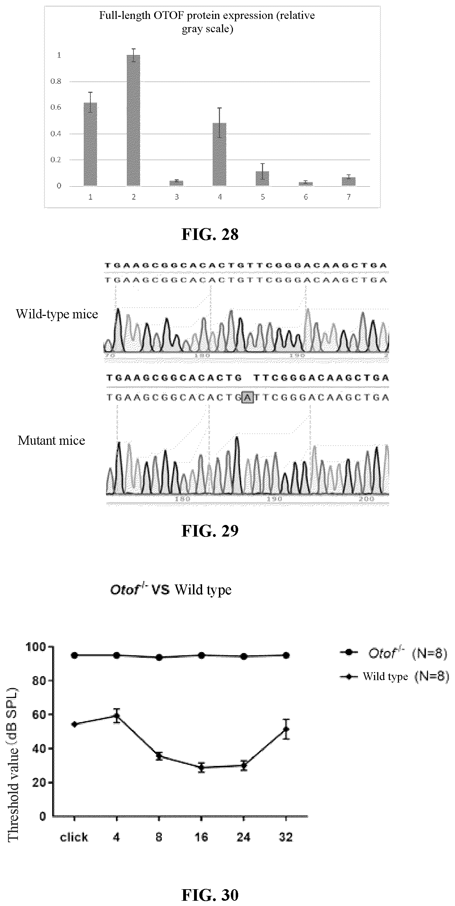

The viruses packaged in example 5 were added into 400 µL of a serum-free DMEM medium (shown in Table 8) in pairs, the materials were mixed evenly, the mixture was added into HEK-293T cells in a 6-well plate (about 1×10⁶ cells and the culture medium was absorbed before the viruses were added), and after the cells were incubated for 4 h, 1.6 mL of DMEM medium containing 10% fetal bovine serum was added. After the cells were cultured for two days, a cell culture medium was absorbed, 200 µL of a cell lysis buffer (1% triton-X 100, 50 mM of Tris-Hcl, and 1 mM of PMSF, pH = 7.4) was added, the cells were placed on ice for 10 min, a cell lysate was collected and centrifuged at 12,000 g and 4 °C for 10 min, and a supernatant was kept. 1/4 volume of a 5 × loading buffer (0.25 M of Tris HCl, 10% SDS, 0.05% bromophenol blue, 50% glycerin, and 0.25 M DTT) was added into the supernatant and the mixture was heated at 90°C for 10 min to obtain a protein sample. 50 µL of the protein sample was subjected to a polyacrylamide gel electrophoresis. After the protein was transferred from gel to a PVDF membrane, aiming at an OTOF antibody (article No. A20266, abclonal), the protein was incubated after diluted at a ratio of 1:3,000 and with the corresponding HRP coupled secondary antibody for 1 h. An ECL reagent was added for imaging. The experiment was repeated for three times. Grey statistics for full-length bands were performed. The results were shown in FIG. 28. It can be seen that the recombination efficiency of the protein by using an OTOF intein recombination method in the present disclosure was obviously higher than the OTOF DNA recombination.

**Table 8 Protein and nucleic acid recombination system**

| Number | Adeno-associated viruses | Viral load |
|---|---|---|
| 1 | pAAV-CMV-OTOF-N-S1-Rma-N-intein | 1×10¹¹ virus genomes |
| | pAAV-CMV-Rma-C-intein-OTOF-C-S1 | 1×10¹¹ virus genomes |
| 2 | pAAV-CMV-OTOF-N-S2-Rma-N-intein | 1×10¹¹ virus genomes |
| | pAAV-CMV-Rma-C-intein-OTOF-C-S2 | 1×10¹¹ virus genomes |
| 3 | pAAV-CMV-OTOF-N-S3-Rma-N-intein | 1×10¹¹ virus genomes |
| | pAAV-CMV-Rma-C-intein-OTOF-C-S3 | 1 × 10¹¹ virus genomes |
| 4 | pAAV-CMV-OTOF-N-S4-Rma-N-intein | 1 ×10¹¹ virus genomes |
| | pAAV-CMV-Rma-C-intein-OTOF-C-S4 | 1 ×10¹¹ virus genomes |
| 5 | pAAV-CMV-OTOF-N-AK | 1 ×10¹¹ virus genomes |
| | pAAV-AK-OTOF-C-PloyA | 1 ×10¹¹ virus genomes |
| 6 | pAAV-CMV-OTOF-N-AP | 1 ×10¹¹ virus genomes |
| | pAAV-AP-OTOF-C-PloyA | 1 ×10¹¹ virus genomes |
| 7 | pAAV-CMV-OTOF-N-TS | 1 ×10¹¹ virus genomes |
| | pAAV-TS-OTOF-C-PloyA | 1 ×10¹¹ virus genomes |

### Example 12 Construction of Otof gene-deficient mice

*Otof*^{*-*/*-*} gene mutant mice were constructed on the basis of 129S2/SvPasCrl strain mice by a CRISPR/Cas9 method. *Otof*^{*-*/*-*} model mice carrying a homozygous mutation with an *OTOF* gene frameshift caused by an *Otof* gene NM_001100395.1:c.2503_2504insA. The results were shown in FIG. 29. A single-base insertion existed in the *Otof*^{*-*/*-*} gene mutant mice to cause a frame shift. The *Otof*^{*-*/*-*} mutant mice and wild-type mice were detected by ABR. The hearing comparison was shown in FIG. 30. The hearing of the mutant mice was completely lost in both ears.

### Example 13 Unilateral ear administration and effect of S1 intein in infant mice

A pAAV-CMV-OTOF-N-S1-Rma-N-intein plasmid and a pAAV-CMV-Rma-C-intein-OTOF-C-S1 plasmid were respectively along with a pHelper plasmid and a pRC plasmid of PHP.eB at a molar ratio of 1:1:1 were co-transfected into HEK-293T cells with a PEI transfection reagent (1 µg of the plasmids were added into about every million cells), the cells were cultured by using a DMEM medium containing 10% fetal bovine serum at a 5% carbon dioxide incubator at 37°C for 3 days, washed once with a PBS buffer, collected, and repeatedly frozen and thawed for five times, a solid NaCl was added to make a final concentration 500 mM, the cells were centrifuged at 10,000 g for half an hour, the obtained supernatant was taken and filtered with a 0.45- µ m filter membrane, a gradient iodixanol solution was prepared and added into a centrifuge tube (5 mL of 60% iodixanol, 5 mL of 40% iodixanol, 6 mL of 25% iodixanol, and 8 mL of 15% iodixanol), the sample was added to an uppermost layer and centrifuged at 350,000 g for 1 h, and a virus layer of 40% and 60% of an interface was sucked. The viruses were centrifuged with a 50 Kda ultrafiltration tube at 10,000 g and treated with a 0.01% poloxamer PBS buffer for 5 times, after a virus titer was measured by using a qPCR method, the tilter was adjusted, finally the viruses with the virus titer of 1±0.2×10¹³ virus genomes/ml, namely pAAV-CMV-OTOF-N-S1-Rma-N-intein PHP. eB AAV and pAAV-CMV-Rma-C-intein-OTOF-C-S1 PHP. eB AAV were obtained, and the solvent is the 0.01% poloxamer PBS buffer.

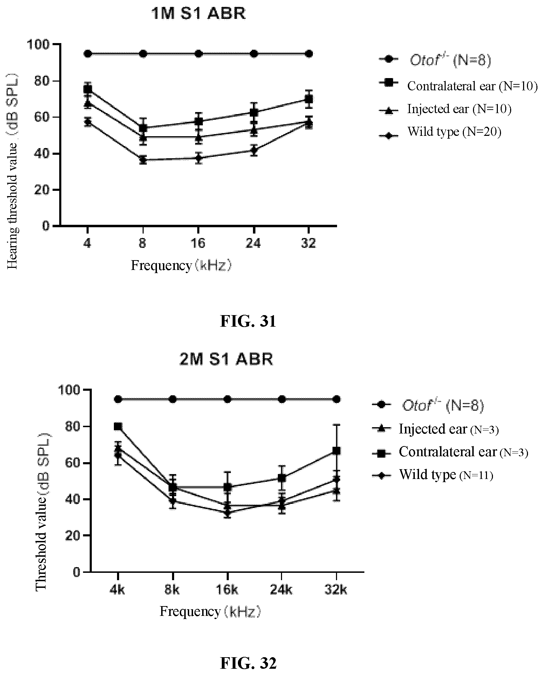

The above constructed adeno-associated viruses were administered to the P0/P1 *Otof*^{*-*/-}gene mutant mice constructed in example 12, specifically, the right cochlea was injected through the round window, and each mouse was administrated with 2×10¹⁰ virus genomes each time (1×10¹⁰ virus genomes of the two AAVs respectively), and an ABR index of the mice was detected to confirm the hearing recovery of the mice. The results after one month were shown in FIG. 31. The round mark in the figure was a non-administration group (n = 8), the square mark was an administration contralateral ear group (n = 10), the triangle mark was an administration ear group (n = 10), and the oblique square mark was a wild-type animal group (n = 20). The results after two months were shown in FIG. 32. The round mark in the figure was the non-administration group (n = 8), the square mark was the contralateral ear group (n = 3), the triangle mark was the administration ear group (n = 3), and the oblique square mark was the wild-type animal group (n = 11). It can be seen that after the AAVs constructed by the present disclosure were used for unilateral cochlear administration of the *Otof*^{*-*/*-*} model mice, the AAVs can restore the hearing of the injection-side ear and also restore the hearing of the contralateral non-injected ear.

### Example 14 Unilateral ear administration and effect of S2 intein in infant mice

A pAAV-CMV-OTOF-N-S2-Rma-N-intein plasmid and a pAAV-CMV-Rma-C-intein-OTOF-C-S2 plasmid were respectively along with a pHelper plasmid and a pRC plasmid of PHP.eB at a molar ratio of 1:1:1 were co-transfected into HEK-293T cells with a PEI transfection reagent (1 µg of the plasmids were added into about every million cells), the cells were cultured by using a DMEM medium containing 10% fetal bovine serum at a 5% carbon dioxide incubator at 37°C for 3 days, washed once with a PBS buffer, collected, and repeatedly frozen and thawed for five times, a solid NaCl was added to make a final concentration 500 mM, the cells were centrifuged at 10,000 g for half an hour, the obtained supernatant was taken and filtered with a 0.45- µ m filter membrane, a gradient iodixanol solution was prepared and added into a centrifuge tube (5 mL of 60% iodixanol, 5 mL of 40% iodixanol, 6 mL of 25% iodixanol, and 8 mL of 15% iodixanol), the sample was added to an uppermost layer and centrifuged at 350,000 g for 1 h, and a virus layer of 40% and 60% of an interface was sucked. The viruses were centrifuged with a 50 Kda ultrafiltration tube at 10,000 g and treated with a 0.01% poloxamer PBS buffer for 5 times, after a virus titer was measured by using a qPCR method, the tilter was adjusted, finally the adeno-associated viruses with the virus titer of 1±0.2×10¹³ virus genomes/ml and an empty shell rate of about 50%, namely pAAV-CMV-OTOF-N-S2-Rma-N-intein PHP.eB AAV and pAAV-CMV-Rma-C-intein-OTOF-C-S2 PHP.eB AAV were obtained, and the solvent is the 0.01% poloxamer PBS buffer.

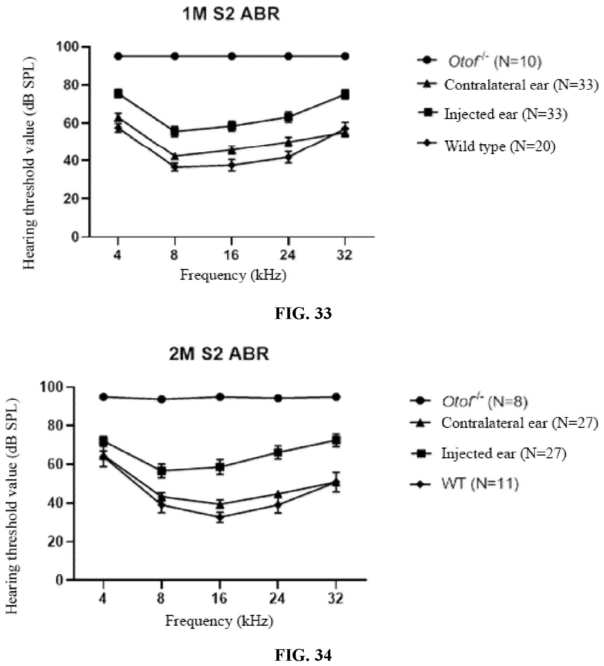

The above constructed adeno-associated viruses were administered to the P0/P1 *Otof*^{*-*/-}gene mutant mice constructed in example 12, specifically, the right cochlea was injected through the round window, and each mouse was administrated with 2×10¹⁰ virus genomes (1×10¹⁰ virus genomes of the two AAVs respectively), and an ABR index of the mice was detected to confirm the hearing recovery of the mice. The results after one month were shown in FIG. 33. The round mark in the figure was a non-administration group (n = 10), the square mark was an administration contralateral ear group (n = 33), the triangle mark was an administration ear group (n = 33), and the oblique square mark was a wild-type animal group (n = 20). The results after two months were shown in FIG. 34. The round mark in the figure was the non-administration group (n = 8), the square mark was the administration contralateral ear group (n = 27), the triangle mark was the administration ear group (n = 27), and the oblique square mark was the wild-type animal group (n = 11). It can be seen that after the AAVs constructed by the present disclosure were used for unilateral cochlear administration of the *Otof*^{*-*/*-*} mutant mice, the AAVs can restore the hearing of the injection-side ear (triangle mark) and also restore the hearing of the contralateral non-injected ear (square mark).

### Example 15 Safety observation of administration group

After the cochlea of 6-8-week-old wild-type CD-1 mice was injected with the AAVs constructed in examples 13 and 14 of the present disclosure through the round window, the daily activities, hair smoothness and food intake of the mice were observed within 3 months. No significant differences in the daily activities, hair smoothness and food intake were found between an administration group and a control group without AAV injection. Besides, no difference was found in hearing between the administration group and the control group. The cochlea of the mice on the 28th and 91st was dissected and inner ear hair cells were subjected to immunofluorescence staining. There was no significant difference in the number of hair cells between the administration group and the control group.

### Example 16 Unilateral ear administration and effect of low-dose drug

A pAAV-CMV-OTOF-N-S2-Rma-N-intein plasmid and a pAAV-CMV-Rma-C-intein-OTOF-C-S2 plasmid were respectively along with a pHelper plasmid and a pRC plasmid of PHP.eB at a molar ratio of 1:1:1 were co-transfected into HEK-293T cells with a PEI transfection reagent (1 µg of the plasmids were added into about every million cells), the cells were cultured by using a DMEM medium containing 10% fetal bovine serum at a 5% carbon dioxide incubator at 37°C for 3 days, washed once with a PBS buffer, collected, and repeatedly frozen and thawed for five times, a solid NaCl was added to make a final concentration 500 mM, the cells were centrifuged at 10,000 g for half an hour, the obtained supernatant was taken and filtered with a 0.45-µm filter membrane, a gradient iodixanol solution was prepared and added into a centrifuge tube (5 mL of 60% iodixanol, 5 mL of 40% iodixanol, 6 mL of 25% iodixanol, and 8 mL of 15% iodixanol), the sample was added to an uppermost layer and centrifuged at 350,000 g for 1 h, and a virus layer of 40% and 60% of an interface was sucked. The viruses were centrifuged with a 50 Kda ultrafiltration tube at 10,000 g and treated with a 0.01% poloxamer PBS buffer for 5 times, after a virus titer was measured by using a qPCR method, the tilter was adjusted, and finally the viruses with the virus titer of 1±0.2× 10¹³ virus genomes/ml, namely pAAV-CMV-OTOF-N-S2-Rma-N-intein PHP.eB AAV and pAAV-CMV-Rma-C-intein-OTOF-C-S2 PHP.eB AAV were obtained. The solvent is the 0.01% poloxamer PBS buffer.

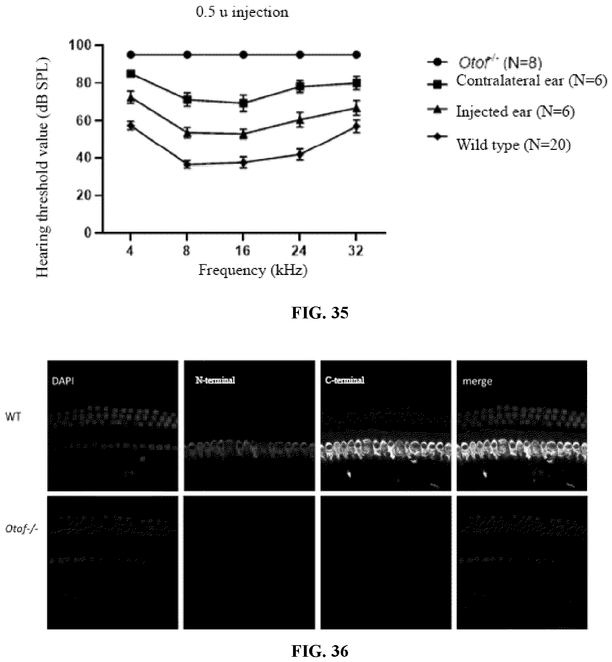

The above constructed adeno-associated viruses were administered to the *Otof*^{*-*/*-*} gene mutant mice constructed in example 12, specifically, the right cochlea was injected through the round window, and each mouse was administrated with 5×10⁹ virus genomes each time (2.5×10⁹ virus genomes of the two AAVs respectively), and an ABR index of the mice was detected to confirm the hearing recovery of the mice. The results after one month were shown in FIG. 35. It can be seen that after the AAVs constructed by the present disclosure were used for unilateral cochlear administration of the *Otof*^{*-*/*-*} model mice, the AAVs can restore the hearing of the injection-side ear and also restore the hearing of the contralateral non-injected ear.

### Example 17 In-vivo protein immunofluorescence assay after administration

The mice 1 month after administration in example 14 were sacrificed, and the cochlear tissue was peeled off and soaked overnight with 4% paraformaldehyde at 4 °C and decalcified with a 10% EDTA solution for three days. Before staining, the tissue was incubated with a 0.3% Triton X-100 PBS buffer for 10 min and blocked with 10% donkey blood for one hour at a room temperature. The tissue was incubated with anti-OTOF antibodies PA5-52935 (C-terminal) or ab53233 (N-terminal) overnight at 4°C and washed three times with 0.1% Triton X-100 PBS for 10 min each time. After the tissue was incubated with a corresponding fluorescent secondary antibody and DAPI for 1 hour, the tissue was washed three times with 0.1%Triton X-100 PBS for 10 min each time. Finally, imaging was performed with a confocal fluorescence microscopy. The results were as follows:
FIG. 36 showed an expression of OTOF in the cochlea of the wild-type mice and *Otof*^{-/-}gene mutant mice. It can be seen from the figure that single-row DAPI-stained cells were inner ear hair cells. It can be seen that the OTOF was significantly expressed in the inner ear hair cells of the wild-type mice and not expressed at all in the *Otof*^{*-*/*-*} gene mutant mice.

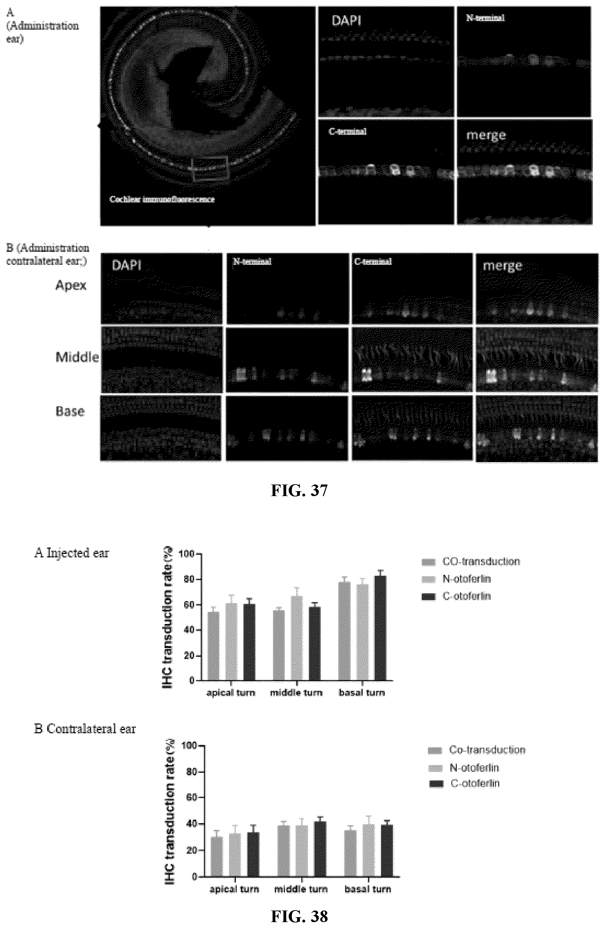

FIG. 37 showed expressions of OTOF in the injected ear and the contralateral ear. A showed an expression of an OTOF protein in the administrated ear of the *Otof*^{*-*/*-*} mice, the left figure was a stretched preparation of the whole cochlea, and the right figure was a partial enlarged view in the left side frame. B showed an expression of an OTOF protein in the administration contralateral ear of the *Otof*^{*-*/*-*} mice, where Apex, Middle, and Base in the figure refer to different regions of the cochlear hair cells. The figure showed that the OTOF was expressed in most of the inner ear hair cells of the administrated ear and the OTOF was also obviously expressed in the administration contralateral ear.

FIG. 38 was statistics of an expression of OTOF in the *Otof*^{*-*/*-*} mice after administration. A was a statistic of inner ear hair cells expressing OTOF in the administrated ear and the number of the inner ear hair cells expressing the OTOF protein was over 60%. B was a statistic of inner ear hair cells expressing OTOF in the administration contralateral ear and the number of the inner ear hair cells expressing the OTOF protein was about 40%. In the figure, apical turn, middle turn, and basal turn referred to different parts of the cochlea. A y-axis ordinate was a transduction rate of the inner ear hair cells.

### Example 18 Unilateral ear administration and effect of four-week-old mice

A pAAV-CMV-OTOF-N-S2-Rma-N-intein plasmid and a pAAV-CMV-Rma-C-intein-OTOF-C-S2 plasmid were respectively along with a pHelper plasmid and a pRC plasmid of AAV1 at a molar ratio of 1:1:1 were co-transfected into HEK-293T cells with a PEI transfection reagent (1 µg of the plasmids were added into about every million cells), the cells were cultured by using a DMEM medium containing 10% fetal bovine serum at a 5% carbon dioxide incubator at 37°C for 3 days, washed once with a PBS buffer, collected, and repeatedly frozen and thawed for five times, a solid NaCl was added to make a final concentration 500 mM, the cells were centrifuged at 10,000 g for half an hour, the obtained supernatant was taken and filtered with a 0.45-µm filter membrane, a gradient iodixanol solution was prepared and added into a centrifuge tube (5 mL of 60% iodixanol, 5 mL of 40% iodixanol, 6 mL of 25% iodixanol, and 8 mL of 15% iodixanol), the sample was added to an uppermost layer and centrifuged at 350,000 g for 1 h, and a virus layer of 40% and 60% of an interface was sucked. The viruses were centrifuged with a 50 Kda ultrafiltration tube at 10,000 g and treated with a 0.01% poloxamer PBS buffer for 5 times, after a virus titer was measured by using a qPCR method, the tilter was adjusted, finally the adeno-associated viruses with the virus titer of 1±0.2× 10¹³ virus genomes/ml and an empty shell rate of about 50%, namely pAAV-CMV-OTOF-N-S2-Rma-N-intein AAV1 and pAAV-CMV-Rma-C-intein-OTOF-C-S2 AAV1 were obtained, and the solvent is the 0.01% poloxamer PBS buffer.

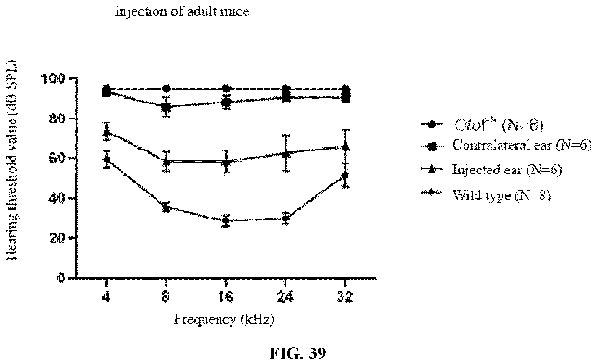

The above constructed adeno-associated viruses were administered to the *Otof*^{*-*/*-*} gene mutant mice (four week old) constructed in example 12, specifically, the right cochlea was injected through the semicircular canal, and each mouse was administrated with 2×10¹⁰ virus genomes (1×10¹⁰ virus genomes of the two AAVs respectively), and an ABR index of the mice was detected to confirm the hearing recovery of the mice. The results after one month were shown in FIG. 39. The round mark in the figure was a non-administration group (n = 8), the square mark was an administration contralateral ear group (n = 6), the triangle mark was an administration ear group (n = 6), and the oblique square mark was a wild-type animal group (n = 8). The results showed that the adeno-associated viruses obtained by the packaging of the present disclosure also had a good effect on the hearing recovery of adult mice and at the same time had a performance of a unilateral ear injection and a bilateral ear recovery.

The above examples are merely illustrative of several implementations of the present disclosure, and the description thereof is more specific and detailed. However, these examples may not to be construed as a limitation to the scope of the patent. It should be noted that those of ordinary skill in the art can further make several variations, combinations and improvements without departing from the conception of the present disclosure. These variations, combinations and improvements all fall within the protection scope of the patent. Therefore, the protection scope of the patent shall be in accordance with the claims.

## Claims

1. A dual-vector system for expressing an OTOF protein, comprising a first nucleotide sequence and a second nucleotide sequence,
wherein the first nucleotide sequence comprises two first ITR sequences and an expression cassette inserted between the two first ITR sequences;
the second nucleotide sequence comprises two second ITR sequences and an expression cassette inserted between the two second ITR sequences;
the expression cassette of the first nucleotide sequence comprises a promoter, an N-terminal coding sequence of OTOF, an N-terminal coding sequence of intein, and a polyA;
the expression cassette of the second nucleotide sequence comprises a promoter, a C-terminal coding sequence of intein, a C-terminal coding sequence of OTOF, and a poly A; and
an OTOF cleavage site is arranged into an OTOF amino acid sequence, the N-terminal coding sequence of the OTOF is a nucleotide coding sequence from an N-terminal of the OTOF amino acid sequence to the OTOF cleavage site, and the C-terminal coding sequence of OTOF is a nucleotide coding sequence from an amino acid next to the OTOF cleavage site to a C-terminal of the OTOF amino acid sequence.

2. The dual-vector system for expressing the OTOF protein according to claim 1, wherein the OTOF amino acid sequence is shown in SEQ ID NO: 1 or SEQ ID NO: 2.

3. The dual-vector system for expressing the OTOF protein according to claim 1, wherein the OTOF cleavage site comprises a preceding amino acid of a serine, a threonine, or a cysteine in the OTOF amino acid sequence.

4. The dual-vector system for expressing the OTOF protein according to claim 1, wherein the promoter of the expression cassette of the first nucleotide sequence or the second nucleotide sequence comprises a CAG promoter, a CMV promoter, a CBA promoter, a UbC promoter, an SFFV promoter, an EF1α promoter, a PGK promoter, or promoters for encoding genes *Myo7A, Myol5, Atohl, POU4F3, Lhx3, Myo6, α9AchR, α10AchR,* and *OTOF*;
the polyA of the expression cassette of the first nucleotide sequence or the second nucleotide sequence comprises AATAAA and variants of the AATAAA, the variants of the AATAAA comprises ATTAAA, AGTAAA, CATAAA, TATAAA, GATAAA, ACTAAA, AATATA, AAGAAA, AATAAT, AAAAAA, AATGAA, AATCAA, AACAAA, AATCAA, AATAAC, AATAGA, AATTAA, or AATAAG; and
each ITR sequence of the two first ITR sequences and the two second ITR sequences is derived from AAV1, AAV2, AAV3, AAV4, AAV5, AAV6, AAV7, AAV8, or AAV9.

5. The dual-vector system for expressing the OTOF protein according to claim 1, wherein the expression cassette of the first nucleotide sequence or the second nucleotide sequence further comprises an expression regulatory element or a tag element.

6. The dual-vector system for expressing the OTOF protein according to claim 1, wherein the intein is derived from MxeGyrA, pabPolIII, MjaKlbA, SspDnaB, SceVMA, SspDnaE, NpuDnaE, AvaDnaE, CraDnaE, CspDnaE, CwaDnaE, MchtDnaE, OliDnaE, TerDnaE, gp41-1, gp41-8, IMPDH-1, or RmaDnaB.

7. The dual-vector system for expressing the OTOF protein according to claim 1, wherein the first nucleotide sequence is an expression cassette inserting the first nucleotide sequence in a plasmid comprising an ITR and between the two first ITR sequences; and the second nucleotide sequence is an expression cassette inserting the second nucleotide sequence in a plasmid comprising an ITR and between the two second ITR sequences.

8. The dual-vector system for expressing the OTOF protein according to claim 7, wherein the plasmid comprising the ITR is a pAAV, pAAV-CMV, pX601, pX551 or pAAV-MCS plasmid.

9. The dual-vector system for expressing the OTOF protein according to claim 1, wherein an 827th amino acid of the OTOF amino acid sequence shown in SEQ ID NO: 2 is used as the OTOF cleavage site and an NpuDnaE Intein is used; after the N-terminal coding sequence of the OTOF and an N-terminal coding sequence of the NpuDnaE Intein are connected and fused, the first nucleotide sequence is constructed with a pAAV-CMV plasmid as a vector; and after a C-terminal coding sequence of the NpuDnaE Intein and the C-terminal coding sequence of the OTOF are connected and fused, the second nucleotide sequence is constructed with the pAAV-CMV plasmid as a vector; or
a 930th amino acid of the OTOF amino acid sequence shown in SEQ ID NO: 2 is used as the OTOF cleavage site and the NpuDnaE Intein is used; after the N-terminal coding sequence of the OTOF and the N-terminal coding sequence of the NpuDnaE Intein are connected and fused, the first nucleotide sequence is constructed with the pAAV-CMV plasmid as a vector; and after the C-terminal coding sequence of the NpuDnaE Intein and the C-terminal coding sequence of the OTOF are connected and fused, the second nucleotide sequence is constructed with the pAAV-CMV plasmid as a vector; or
a 1,130th amino acid of the OTOF amino acid sequence shown in SEQ ID NO: 2 is used as the OTOF cleavage site and the NpuDnaE Intein is used; after the N-terminal coding sequence of the OTOF and the N-terminal coding sequence of the NpuDnaE Intein are connected and fused, the first nucleotide sequence is constructed with the pAAV-CMV plasmid as a vector; and after the C-terminal coding sequence of the NpuDnaE Intein and the C-terminal coding sequence of the OTOF are connected and fused, the second nucleotide sequence is constructed with the pAAV-CMV plasmid as a vector; or
an 827th amino acid of the OTOF amino acid sequence shown in SEQ ID NO: 2 is used as the OTOF cleavage site and a RmaDnaB Intein is used; after the N-terminal coding sequence of the OTOF and a N-terminal coding sequence of the RmaDnaB Intein are connected and fused, the first nucleotide sequence is constructed with the pAAV-CMV plasmid as a vector; and after a C-terminal coding sequence of the RmaDnaB Intein and the C-terminal coding sequence of the OTOF are connected and fused, the second nucleotide sequence is constructed with the pAAV-CMV plasmid as a vector; or
a 930th amino acid of the OTOF amino acid sequence shown in SEQ ID NO: 2 is used as the OTOF cleavage site and the RmaDnaB Intein is used; after the N-terminal coding sequence of the OTOF and the N-terminal coding sequence of the RmaDnaB Intein are connected and fused, the first nucleotide sequence is constructed with the pAAV-CMV plasmid as a vector; and after the C-terminal coding sequence of the RmaDnaB Intein and the C-terminal coding sequence of the OTOF are connected and fused, the second nucleotide sequence is constructed with the pAAV-CMV plasmid as a vector; or
a 954th amino acid of the OTOF amino acid sequence shown in SEQ ID NO: 2 is used as the OTOF cleavage site and the RmaDnaB Intein is used; after the N-terminal coding sequence of the OTOF and the N-terminal coding sequence of the RmaDnaB Intein are connected and fused, the first nucleotide sequence is constructed with the pAAV-CMV plasmid as a vector; and after the C-terminal coding sequence of the RmaDnaB Intein and the C-terminal coding sequence of the OTOF are connected and fused, the second nucleotide sequence is constructed with the pAAV-CMV plasmid as a vector; or
a 1,130th amino acid of the OTOF amino acid sequence shown in SEQ ID NO: 2 is used as the OTOF cleavage site and the RmaDnaB Intein is used; after the N-terminal coding sequence of the OTOF and the N-terminal coding sequence of the RmaDnaB Intein are connected and fused, the first nucleotide sequence is constructed with the pAAV-CMV plasmid as a vector; and after the C-terminal coding sequence of the RmaDnaB Intein and the C-terminal coding sequence of the OTOF are connected and fused, the second nucleotide sequence is constructed with the pAAV-CMV plasmid as a vector.

10. A packaging vector system for an adeno-associated virus, wherein the packaging vector system comprises the dual-vector system for expressing the OTOF protein according to any one of claims 1-9, a vector carrying AAV *rep* and *cap* genes, and a helper virus vector, and the vector carrying the AAV *rep* and *cap* genes and the helper virus vector are packaged into AAV vectors.

11. The packaging vector system for the adeno-associated virus according to claim 10, wherein the vector carrying the AAV *rep* and *cap* genes is selected from the group consisting of AAV1, AAV2, AAV5, AAV8, AAV9, Anc80, PHP.eB, AAV-DJ, and AAVrh.10 vectors; and the helper virus vector is a pHelper plasmid.

12. A packaging method for an adeno-associated virus, wherein the packaging vector system for the adeno-associated virus according to claim 10 is transferred into a host cell for packaging.

13. The packaging method for the adeno-associated virus according to claim 12, wherein the host cell is selected from the group consisting of a Hela-S3 cell, a HEK-293 cell, a HEK-293T cell, a HEK-293FT cell, a A549 cell, and a Sf9 cell.

14. The adeno-associated virus obtained by the packaging method according to claim 12.

15. A use of the dual-vector system for expressing the OTOF protein according to claim 1 or the adeno-associated virus according to claim 14 in preparing a drug or a preparation for treating a deafness disease or a hearing impairment or a hearing dysfunction.

16. A drug or a preparation for treating a deafness disease or a hearing impairment or a hearing dysfunction prepared from the dual-vector system for expressing the OTOF protein according to claim 1 or the adeno-associated virus according to claim 14.

17. The drug or the preparation according to claim 16, wherein the drug or the preparation further comprises one or more of a neutral salt buffer, an acidic salt buffer, an alkaline salt buffer, glucose, mannose, mannitol, a protein, a polypeptide, an amino acid, an antibiotic, a chelating agent, an adjuvant, a preservative, a nanoparticle, a liposome, and a positive lipid particle.

18. The drug or the preparation according to claim 16, wherein an injection administration is performed through a round window, an oval window, a semicircular canal, and an alveus communis of a cochlea; and
a lifetime single administration or a multiple administration is performed with a total dose of 1×10⁹-1×10¹³ virus genomes.
